(19) Europäisches Patentamt
European Patent Office
Office européen des brevets

(11) Veröffentlichungsnummer: **0 232 538**
**B1**

(12) # EUROPÄISCHE PATENTSCHRIFT

(45) Veröffentlichungstag der Patentschrift: **23.05.90**

(51) Int. Cl.[5]: **C07D 231/40**, C07D 401/04, A01N 43/56, A01N 43/40

(21) Anmeldenummer: **86117970.3**

(22) Anmeldetag: **23.12.86**

(54) **5-Acylamido-1-aryl-pyrazole.**

(30) Priorität: **15.01.86 DE 3600950**

(43) Veröffentlichungstag der Anmeldung: **19.08.87 Patentblatt 87/34**

(45) Bekanntmachung des Hinweises auf die Patenterteilung: **23.05.90 Patentblatt 90/21**

(84) Benannte Vertragsstaaten: **AT BE CH DE ES FR GB IT LI NL SE**

(56) Entgegenhaltungen:
**EP-A- 0 068 806**
**EP-A- 0 154 115**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Gehring, Reinhold, Dr., Dasnöckel 49, D-5600 Wuppertal 11(DE)**
Erfinder: **Schallner, Otto, Dr., Noldeweg 22, D-4019 Monheim(DE)**
Erfinder: **Stetter, Jörg, Dr., Gellertweg 4, D-5600 Wuppertal 1(DE)**
Erfinder: **Santel, Hans-Joachim, Dr., Grünstrasse 9a, D-5090 Leverkusen 1(DE)**
Erfinder: **Schmidt, Robert R., Dr., Im Waldwinkel 110, D-5060 Bergisch-Gladbach 2(DE)**

**EP 0 232 538 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue 5-Acylamido-1-aryl-pyrazole, mehrere Verfahren zu ihrer Herstellung sowie ihre Verwendung als Herbizide.

Es ist bereits bekannt, daß bestimmte 5-Acylamido-1-arylpyrazole, wie beispielsweise das 4-Cyano-5-propionamido-1(2,4,6-trichlorphenyl)-pyrazol herbizide Eigenschaften besitzen (vgl. z.B. DE-OS 3 226 513).

Weiterhin ist bekannt, daß substituierte 5-Amino-1-phenylpyrazole herbizide Eigenschaften besitzen (vgl. EP-A 154 115).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsbereichen völlig zufriedenstellend.

Es wurden neue 5-Acylamido-1-aryl-pyrazole der allgemeinen Formel (I),

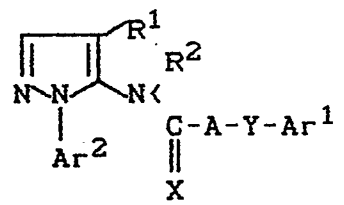

(I)

in welcher

$R^1$ für Nitro, Fluor, Chlor, Brom oder Iod steht,

$R^2$ für Wasserstoff oder für geradkettiges oder verzweigtes Alkyl mit 1 bis 12 Kohlenstoffatomen steht,

X für Sauerstoff oder Schwefel steht,

Y für Sauerstoff, Schwefel, eine Sulfinyl- oder eine Sulfonylgruppe steht,

A für eine geradkettige oder verzweigte, gegebenenfalls einfach oder mehrfach, gleich oder verschieden durch Halogen substituierte Alkylenbrücke mit 1 bis 6 Kohlenstoffatomen steht,

$Ar^1$ für jeweils gegebenenfalls einfach oder mehrfach, gleich oder verschieden substituiertes Phenyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, Pyrazinyl oder s-Triazinyl steht, wobei als Substituenten jeweils in Frage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$,

wobei

$R^4$ für Amino, für jeweils geradkettiges oder verzweigtes Alkyl, Alkylamino, Dyalkylamino oder Halogenalkyl mit jeweils 1 bis 4 Kohlenstoffatomen in den einzelnen Alkylteilen und im Fall des Halogenalkyls mit 1 bis 9 gleichen oder verschiedenen Halogenatomen steht und

m für eine Zahl 0, 1 oder 2 steht sowie

$Ar^2$ für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils die bei $Ar^1$ genannten in Fage kommen,

gefunden.

Weiterhin wurde gefunden, daß man die neuen 5-Acylamido-1aryl-pyrazole der Formel (I) mit Hilfe der im folgenden beschriebenen Verfahren erhält:

a) Man erhält 5-Acylamido-1-aryl-pyrazole der Formel (Ia),

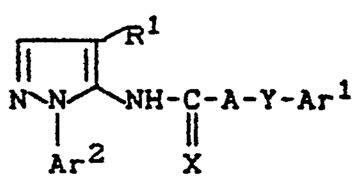

(Ia)

in welcher

$R^1$, X, Y, A, $Ar^1$ und $Ar^2$ die oben angegebene Bedeutung haben,

wenn man 5-Amino-1-aryl-pyrazole der Formel (II),

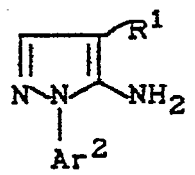

(II)

in welcher
$R^1$ und $Ar^2$ die oben angegebene Bedeutung haben,
mit Acylierungsmitteln der Formel (III),

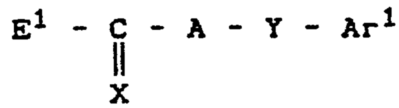

in welcher
X, Y, A und $Ar^1$ die oben angegebene Bedeutung haben und
$E^1$ für eine elektronenanziehende Abgangsgruppe steht,
gegebenenfalls in Gegenwart eines Verdünnungsmittels, gegebenenfalls in Gegenwart eines Säurebindemittels sowie gegebenenfalls in Gegenwart eines Katalysators umsetzt;

b) Man erhält 5-Acylamido-1-aryl-pyrazole der Formel (Ib),

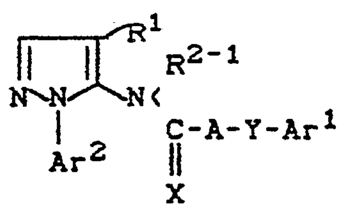

(Ib)

in welcher
$R^1$, X, Y, A, $Ar^1$ und $Ar^2$ die angegebene Bedeutung haben und
$R^{2-1}$ für geradkettiges oder verzweigtes Alkyl mit 1 bis 12 Kohlenstoffatomen steht, wenn man die nach Verfahren (a) erhältlichen 5-Acylamido-1-aryl-pyrazole der Formel (Ia),

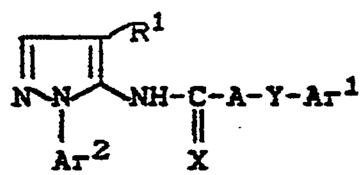

(Ia)

in welcher
$R^1$, X, Y, A, $Ar^1$ und $Ar^2$ die oben angegebene Bedeutung haben,
mit Alkylierungsmitteln der Formel (IV),
$E^2 - R^{2-1}$ (IV)
in welcher
$R^{2-1}$ die oben angegebene Bedeutung hat und
$E^2$ für Halogen, für gegebenenfalls substituiertes Alkoxysulfonyloxy oder für gegebenenfalls substituiertes Arylsulfonyloxy steht,
gegebenenfalls in Gegenwart eines Verdünnungsmittels und gegebenenfalls in Gegenwart eines Säurebindemittels sowie gegebenenfalls in Gegenwart eines Katalysators umsetzt;

c) Man erhält 5-Acylamido-1-aryl-pyrazole der Formel (I), wenn man 5-Acylamido-1-aryl-pyrazole der Formel (V),

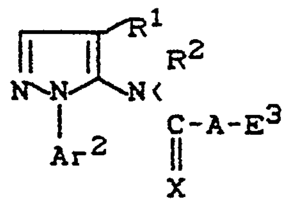

(V)

in welcher
$R^1$, $R^2$, X, A und $Ar^2$ die oben angegebene Bedeutung haben und
$E^3$ für Halogen steht
mit Phenol- bzw. Thiophenolderivaten der Formel (VI),
$Ar^1 - Y - Z$ (VI)
in welcher
$Ar^1$ und Y die oben angegebene Bedeutung haben und
Z für Wasserstoff oder für ein Äquivalent eines Erdalkali- oder Alkalimetallkations steht,
gegebenenfalls in Gegenwart eines Verdünnungsmittels und gegebenenfalls in Gegenwart eines Säurebindemittels und gegebenenfalls in Gegenwart eines geeigneten Katalysators umsetzt;

d) Man erhält 5-Acylamido-1-aryl-pyrazole der Formel (Ic)

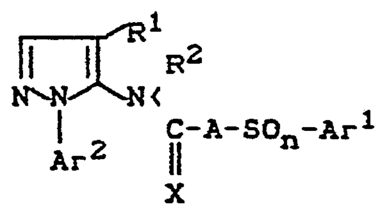

(Ic)

in welcher
$R^1$, $R^2$, X, A, $Ar^1$ und $Ar^2$ die oben angegebene Bedeutung haben und
n für eine Zahl 1 oder 2 steht,
wenn man die mit Hilfe der Verfahren (a), (b) oder (c) erhältlichen 5-Acylamido-1-aryl-pyrazole der Formel (Id),

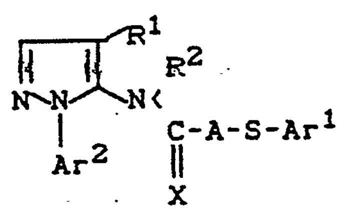

(Id)

in welcher
$R^1$, $R^2$, X, A, $Ar^1$ und $Ar^2$ die oben angegebene Bedeutung haben,
mit Oxidationsmitteln der Formel (VII),
$R^3 - O - O - H$ (VII)
in welcher
$R^3$ für Wasserstoff oder für jeweils gegebenenfalls substituiertes Alkanoyl oder Aroyl steht,
gegebenenfalls in Gegenwart eines Verdünnungsmittels und gegebenenfalls in Gegenwart eines Säurebindemittels sowie gegebenenfalls in Gegenwart eines Katalysators umsetzt;

e) Man erhält 5-Acylamido-1-aryl-pyrazole der Formel (Ie)

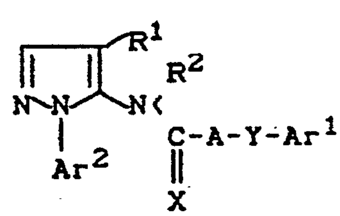

(Ie)

in welcher
$R^2$, X, A, Y, $Ar^1$ und $Ar^2$ die oben angegebene Bedeutung haben und
$R^1$ für Halogen oder Nitro steht,

wenn man die mit Hilfe der Verfahren (a), (b), (c) oder (d) erhältlichen 5-Acylamido-1-aryl-pyrazole der Formel (If),

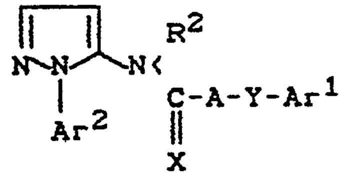

(If)

in welcher
$R^2$, X, Y, A, $Ar^1$ und $Ar^2$ die oben angegebene Bedeutung haben,
mit Halogenierungs- oder Nitrierungsmitteln der Formel (VIII),
$R^1 - E^4$ (VIII)
in welcher
$R^1$ die oben angegebene Bedeutung hat und
$E^4$ für eine elektronenanziehende Abgangsgruppe steht,
gegebenenfalls in Gegenwart eines Verdünnungsmittels und gegebenenfalls in Gegenwart eines Katalysators oder Reaktionshilfsmittels umsetzt.

Schließlich wurde gefunden, daß die neuen 5-Acylamido-1aryl-pyrazole der allgemeinen Formel (I) herbizide insbesondere auch selektiv-herbizide Eigenschaften besitzen.

Überraschenderweise zeigen die erfindungsgemäßen 5-Acylamido-1-aryl-pyrazole der allgemeinen Formel (I) eine erheblich bessere allgemein-herbizide Wirksamkeit gegen schwer bekämpfbare Problemunkräuter und gleichzeitig eine deutlich verbesserte Verträglichkeit gegenüber wichtigen Kulturpflanzen im Vergleich zu den aus dem Stand der Technik bekannten 5-Acylamido-1-aryl-pyrazolen, wie beispielsweise dem 4-Cyano-5-propionamido-1-(2,4,6-trichlorphenyl)-pyrazol, welche chemisch und wirkungsmäßig naheliegende Verbindungen sind.

Die erfindungsgemäßen 5-Acylamido-1-aryl-pyrazole sind durch die Formel (I) allgemein definiert. Bevorzugt sind Verbindungen der Formel (I), bei welchen
$R^1$ für Nitro, Chlor oder Brom steht,
$R^2$ für Wasserstoff oder für geradkettiges oder verzweigtes Alkyl mit 1 bis 8 Kohlenstoffatomen steht,
X für Sauerstoff oder Schwefel steht,
Y für Sauerstoff, Schwefel, eine Sulfinylgruppe oder eine Sulfonylgruppe steht,
A für ein Brückenglied der Formel $-CH_2-$; $-CH_2-CH_2-$;

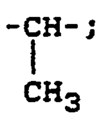; $-CCl_2-CH_2-$; $-CHCl-CH_2-$; 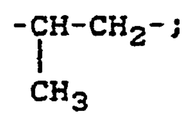; $-CH_2-CH_2-CH_2-$;

$-CH_2-CH_2-CH_2-CH_2-$; 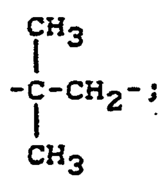; 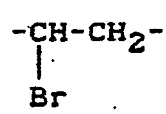 oder $-CBr_2-CH_2-$

steht,
$Ar^1$ für jeweils gegebenenfalls ein- bis dreifach, gleioh oder verschieden substituiertes Phenyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, Pyrazinyl oder s-Triazinyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Di-chlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$, wobei
$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und
m für eine Zahl 0, 1 oder 2 steht sowie

$Ar^2$ für gegebenenfalls ein- bis fünffach, gleich oder verschieden substituiertes Phenyl oder für jeweils gegebenenfalls ein- bis vierfach, gleich oder verschieden substituiertes 2-Pyridyl oder 4-Pyridyl steht, wobei als Substituenten jeweils die bei $Ar^1$ genannten in Frage kommen.

Besonders bevorzugt sind 5-Acylamido-1-aryl-pyrazole der allgemeinen Formel (I), bei welchen

$R^1$ für Nitro steht,

$R^2$ für Wasserstoff, Methyl oder Ethyl steht,

X für Sauerstoff oder Schwefel steht,

Y für Sauerstoff, Schwefel, eine Sulfinylgruppe oder eine Sulfonylgruppe steht,

A für ein Brückenglied der Formel $-CH_2-$; $-CH_2-CH_2-$;

$-CH_2-CH_2-CH_2-$; 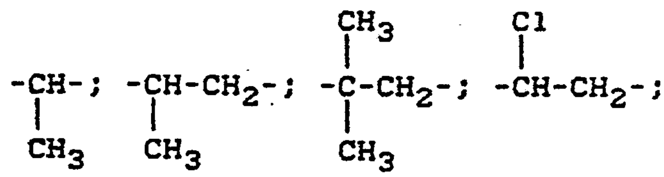

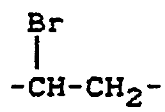 oder $-CCl_2-CH_2-$ steht,

$Ar^1$ für jeweils gegebenenfalls ein- bis dreifach, gleich oder verschieden substituiertes Phenyl, 2-Pyridyl, 4-Pyridyl, 2-Pyrimidyl, 4-Pyrimidyl, 5-Pyrimidyl oder s-Triazinyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder ein Rest $-S(O)_m-R^4$, wobei

$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und

m für eine Zahl 0, 1 oder 2 steht sowie

$Ar^2$ für gegebenenfalls ein- bis fünffach gleich oder verschieden substituiertes Phenyl oder für gegebenenfalls ein- bis vierfach, gleich oder verschieden substituiertes 2-Pyridyl steht, wobei als Substituenten jeweils in Frage kommen: Fluor, Chlor, Brom, Methyl, Ethyl, n- oder i-Propyl, Trifluormethyl, Difluorchlormethyl, Fluordichlormethyl, Trifluormethoxy oder ein Rest $-S(O)_p-R^5$, wobei

$R^5$ für Methyl, Difluorchlormethyl, Dichlorfluormethyl, Trifluormethyl, Amino, Dimethylamino oder Diethylamino steht und

p für eine Zahl 0, 1 oder 2 steht.

Im einzelnen seien außer den bei den Herstellungsbeispielen genannten Verbindungen die folgenden 5-Acylamido-1aryl-pyrazole der allgemeinen Formel (I) genannt:

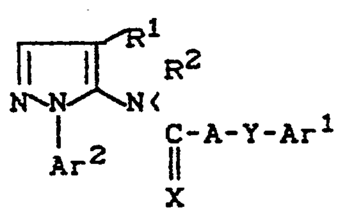 (I)

Tabelle 1

| $R^1$ | $R^2$ | $-\overset{X}{\overset{\|}{C}}-A-Y-Ar^1$ | $Ar^2$ |
|---|---|---|---|
| $NO_2$ | H | 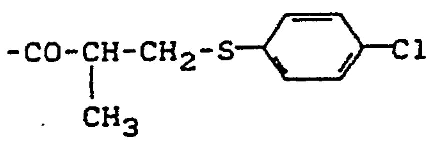 | 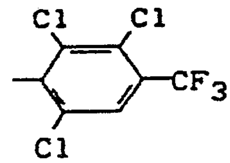 |
| $NO_2$ | $CH_3$ | 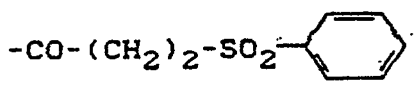 | 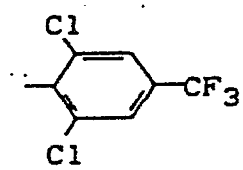 |
| $NO_2$ | H | 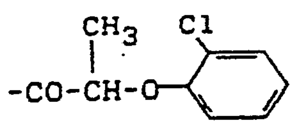 | 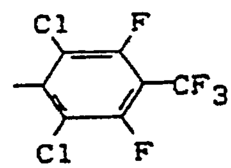 |
| $NO_2$ | H | 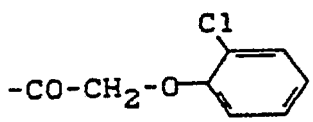 | 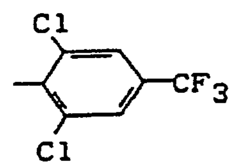 |
| $NO_2$ | H | 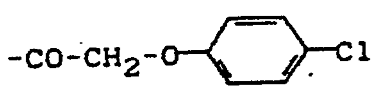 | 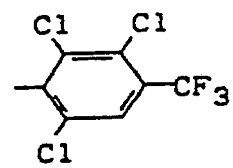 |
| $NO_2$ | H | 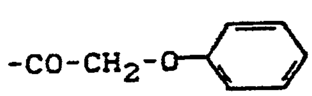 | 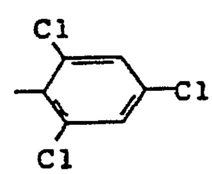 |

Tabelle 1 (Fortsetzung)

| $R^1$ | $R^2$ | X<br>‖<br>$-C-A-Y-Ar^1$ | $Ar^2$ |
|---|---|---|---|
| $NO_2$ | H | 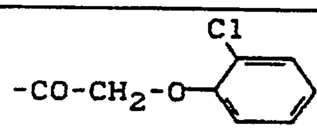 | 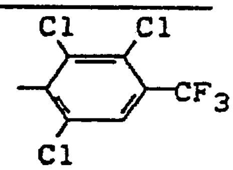 |
| $NO_2$ | H | 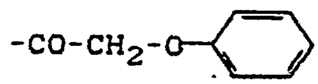 | 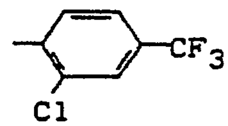 |
| $NO_2$ | H | 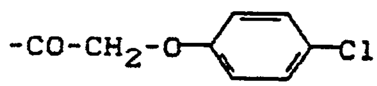 | 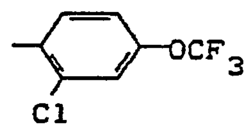 |
| $NO_2$ | H | 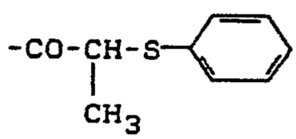 | 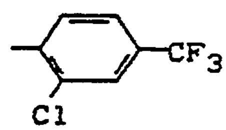 |
| $NO_2$ | H | 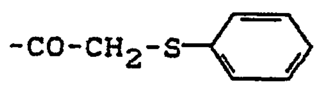 | 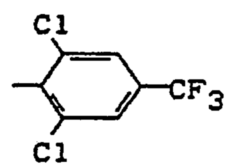 |
| $NO_2$ | H | 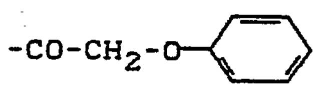 | 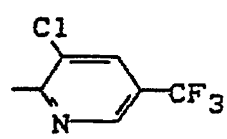 |
| $NO_2$ | H | 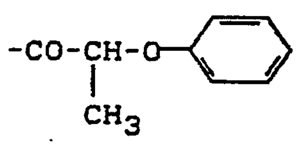 | 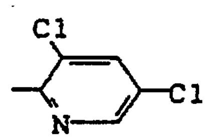 |
| $NO_2$ | H | 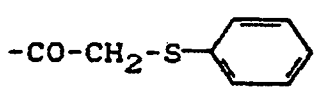 | 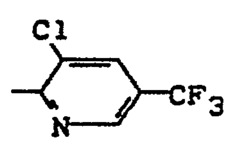 |

Tabelle 1 (Fortsetzung)

| $R^1$ | $R^2$ | $\overset{X}{\overset{\|}{-C}}-A-Y-Ar^1$ | $Ar^2$ |
|---|---|---|---|
| $NO_2$ | H | $-CO-CH_2-SO_2-$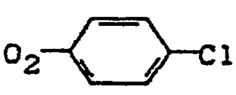 | 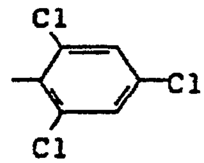 |
| $NO_2$ | H | $-CO-CH_2-CH_2-O-$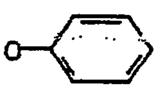 | 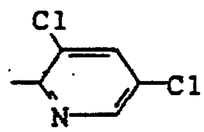 |
| $NO_2$ | H | $-CO-CH_2-CH_2-S-$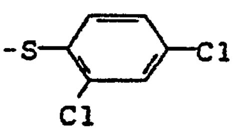 | 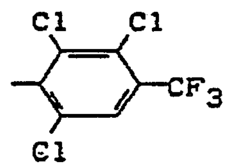 |

Verwendet man bsispielsweise 5-Amino-4-nitro-1-(2,6-di-chlor-4-trifluormethyl-phenyl)-pyrazol und 3-Phenylsulfonylpropionsäurechlorid als Ausgangsstoffe, so läßt sich der Reaktionsablauf des erfindungsgemäßen Verfahrens (a) durch das folgende Formelschema darstellen:

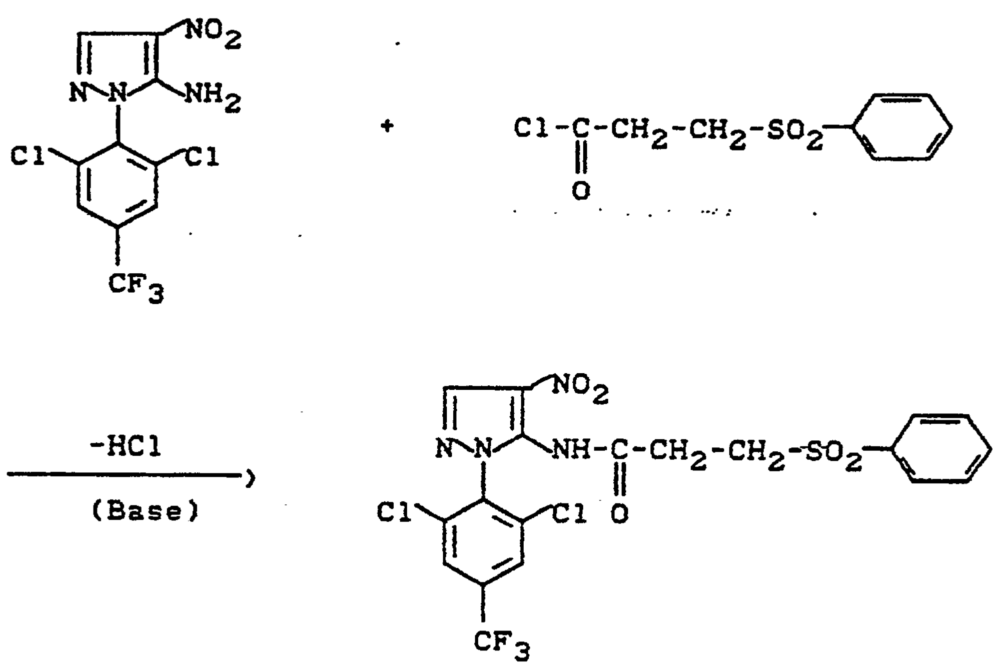


Verwendet man beispielsweise 5-(3-Chlorpropionamido)-4nitro-1-(2-chlor-4-trifluormethyl-phenyl)-pyrazol und Thiophenol als Ausgangsstoffe, so läßt sich der Reaktionsablauf des erfindungsgemäßen Verfahrens (c) durch das folgende Formelschema darstellen:

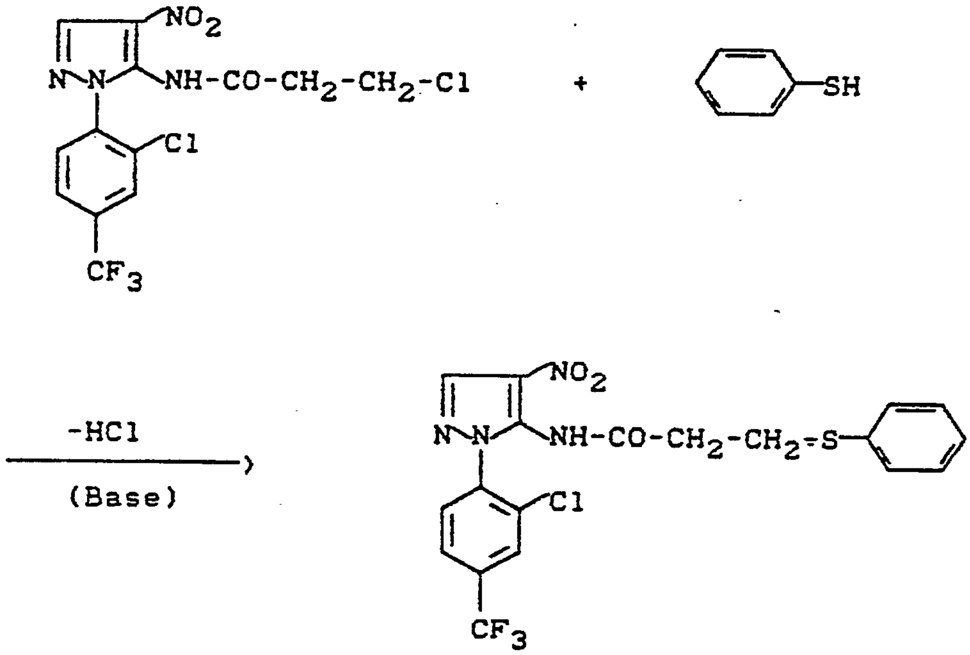


Verwendet man beispielsweise 5-Phenylthioacetamido-4-nitro-1-(2-chlor-4-trifluormethoxy-phenyl)-pyrazol als Ausgangsverbindung und 3-Chlorperbenzoesäure als Oxidationsmittel, so läßt sich der Reaktionsablauf des erfindungsgemäßen Verfahrens (d) durch das folgende Formelschema darstellen:

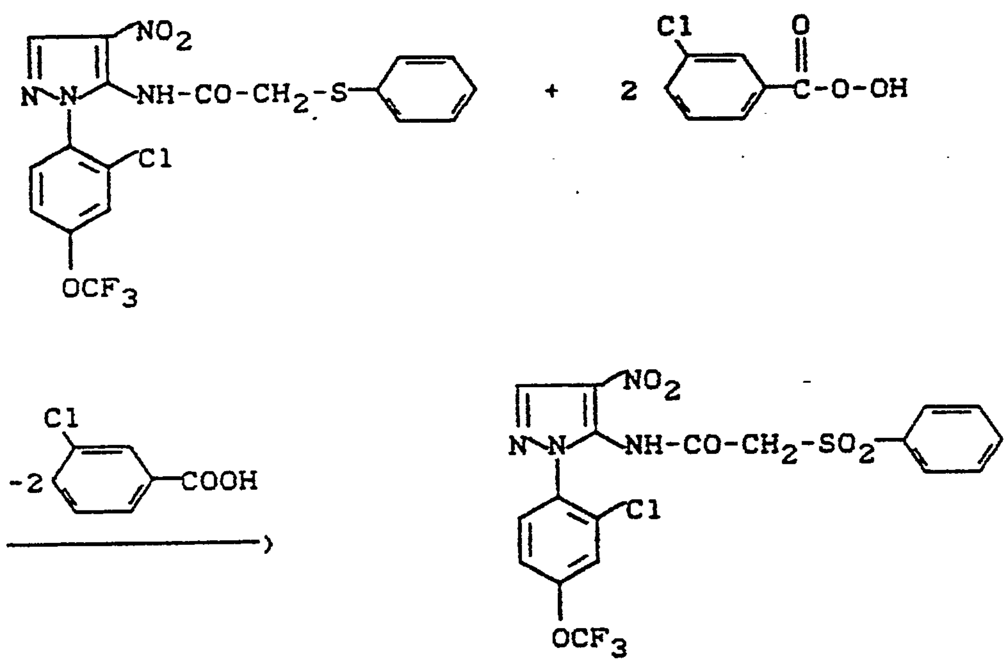

Verwendet man beispielsweise 5-(2-Phenoxypropionamido)-1-(2,6-dichlor-4-trifluormethylthio-phenyl)-pyrazol und Salpetersäure als Ausgangsstoffe, so läßt sich der Reaktionsablauf des erfindungsgemäßen Verfahrens (e) durch das folgende Formelschema darstellen:

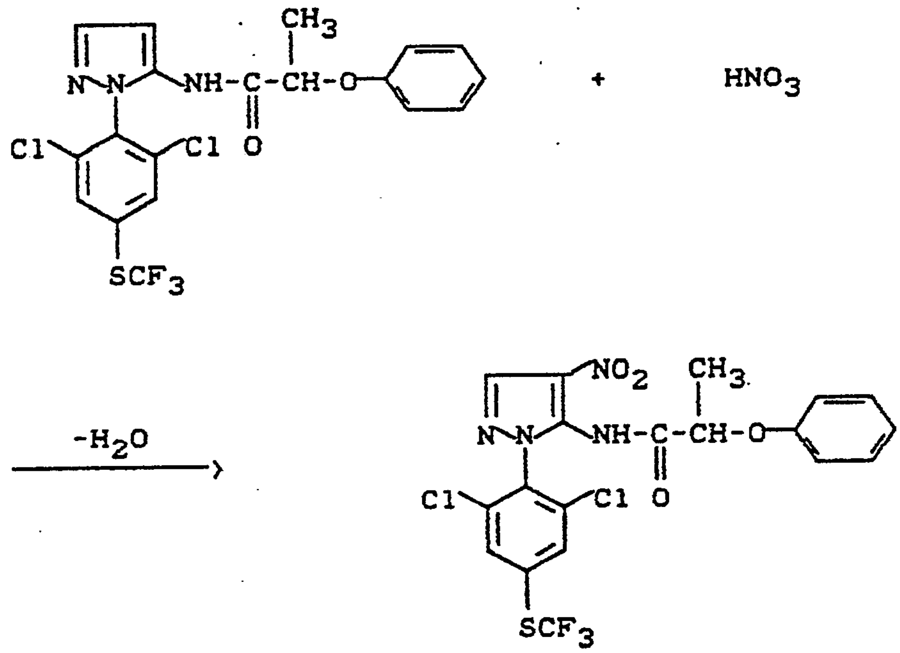


Die zur Durchführung des erfindungsgemäßen Verfahrens (a) als Ausgangsstoffe benötigten 5-Amino-1-aryl-pyrazole sind durch die Formel (II) allgemein definiert. In dieser Formel (II) stehen $R^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Amino-1-aryl-pyrazole der Formel (II) sind teilweise bekannt (vgl. z.B. DE-OS 3 402 308) teilweise sind sie Gegenstand der eigenen vorgängigen noch nicht publizierten Patentanmeldung DE-P 3 520 330 vom 07.06.1985 und erhältlich in Analogie zu bekannten Verfahren (vgl. DE-OS 3 402 308), beispielsweise wenn man Arylhydrazine der Formel (IX),

$Ar^2 - NH - NH^2$ (IX)

in welcher

$Ar^2$ die oben angegebene Bedeutung hat,

mit 2-Halogenacrylnitrilen der Formel (X),

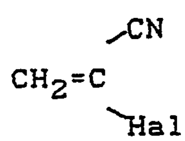 (X)

in welcher

Hal für Halogen, insbesondere für Chlor oder Brom steht,

entweder zunächst in einer 1. Stufe, gegebenenfalls in Gegenwart eines Verdünnungsmittels wie beispielsweise Eisessig oder Ethanol sowie gegebenenfalls in Gegenwart eines Reaktionshilfsmittels wie beispielsweise Natriumacetat bei Temperaturen zwischen –20°C und +20°C umsetzt zu den Arylhydrazin-Derivaten der Formel (XI),

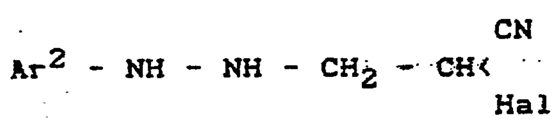 (XI)

in welcher

$Ar^2$ und Hal die oben angegebene Bedeutung haben,

und diese in einer 2. Stufe, gegebenenfalls in Gegenwart eines Verdünnungsmittels wie beispielsweise Ethylenglykolmonoethylether und gegebenenfalls in Gegenwart eines sauren Katalysators wie beispielsweise Schwefelsäure oder Phosphorsäure bei Temperaturen zwischen +50° C und +150° C cyclisiert, oder direkt in einem Reaktionsschritt ohne Isolierung der Zwischenstufe der Formel (XI), gegebenen-

falls in Gegenwart eines Verdünnungsmittels wie beispielsweise Ethylenglykolmonoethylether oder Ethanol bei Temperaturen zwischen +50°C und +150°C cyclisiert und die so erhältlichen 4-unsubstituierten 5-Amino-1-aryl-pyrazole der Formel (IIa),

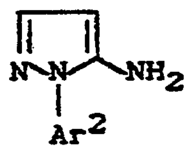

(IIa)

in welcher

$Ar^2$ die oben angegebene Bedeutung hat,

in einer Folgereaktion mit einem Nitrierungsmittel, wie beispielsweise Salpetersäure gegebenenfalls in Gegenwart eines Verdünnungsmittels wie beispielsweise Eisessig und gegebenenfalls in Gegenwart eines Reaktionshilfsmittels wie beispielsweise Acetanhydrid bei Temperaturen zwischen –20°C und +50°C nitriert oder alternativ mit einem Halogenierungsmittel, wie beispielsweise Chlor, Sulfurylchlorid, Phosphorpentachlorid, N-Chlorsuccinimid, Brom, Phosphortribromid oder N-Bromsuccinimid, gegebenenfalls in Gegenwart eines Verdünnungsmittels, wie beispielsweise Methylenchlorid oder Eisessig, und gegebenenfalls in Gegenwart eines Reaktionshilfsmittels wie beispielsweise Bortrifluorid bei Temperaturen zwischen –20°C und +50°C halogeniert.

Dabei kann es gegebenenfalls von Vorteil sein, vor der Halogenierungs- bzw. Nitrierungsreaktion die Aminogruppe in der 5-Position des Pyrazolringes mit Hilfe üblicher Schutzgruppentechnik beispielsweise durch Acylierung zu schützen und die Aminoschutzgruppe nach erfolgter Halogenierung bzw. Nitrierung ebenfalls in üblicher Art und Weise beispielsweise durch Verseifung mit wäßriger oder alkoholischer Base wieder abzuspalten.

Die Arylhydrazine (IX) sind bekannt (vgl. z.B. US-PS 4 127 575; US-PS 3 609 158; DE-OS 2 558 399; J. Chem. Soc. C. 1971, 167–174) oder können nach bekannten Verfahren in einfacher analoger Weise erhalten werden (vgl. z.B. Houben-Weyl «Methoden der organischen Chemie» Band X/2, S. 203, Thieme Verlag Stuttgart 1967; DE-OS 3 402 308).

Die Halogenacrylnitrile der Formel (X) sind allgemein bekannte Verbindungen der organischen Chemie.

Die zur Durchführung des erfindungsgemäßen Verfahrens (a) weiterhin als Ausgangsstoffe benötigten Acylierungsmittel sind durch die Formel (III) allgemein definiert. In dieser Formel (III) stehen X, Y, A und $Ar^1$ vorzugsweise für diejenigen Reste, die bereits im Zusammenhang mit der Beschreibung der erfindungsgemäßen Stoffe der Formel (I) als bevorzugt für diese Substituenten genannt wurden.

$E^1$ steht vorzugsweise für Halogen, insbesondere Chlor oder Brom oder für einen Rest

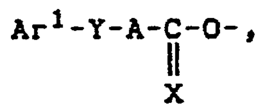

wobei X, Y, A und $Ar^1$ die oben angegebene Bedeutung haben. Die Acylierungsmittel der Formel (III) sind allgemein bekannte Verbindungen der organischen Chemie.

Die zur Durchführung des erfindungsgemäßen Verfahrens (b) als Ausgangsstoffe benötigten 5-Acylamido-1-aryl-pyrazole sind durch die Formel (Ia) allgemein definiert. In dieser Formel (Ia) stehen $R^1$, X, Y, A, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Acylamido-1-aryl-pyrazole der Formel (Ia) sind erfindungsgemäße Verbindungen und erhältlich mit Hilfe der erfindungsgemäßen Verfahren (a), (c), (d) oder (e).

Die zur Durchführung des erfindungsgemäßen Verfahrens (b) weiterhin als Ausgangsstoffe benötigten Alkylierungsmittel sind durch die Formel (IV) allgemein definiert. In dieser Formel (IV) steht $R^{2-1}$ vorzugsweise für Methyl, Ethyl, n- oder i-Propyl sowie n-, i, s- oder t-Butyl.

$E^2$ steht vorzugsweise für Chlor, Brom oder Iod, für Methoxysulfonyloxy oder für p-Toluolsulfonyloxy.

Die Alkylierungsmittel der Formel (IV) sind allgemein bekannte Verbindungen der organischen Chemie.

Die zur Durchführung des erfindungsgemäßen Verfahrens (c) als Ausgangsstoffe benötigten 5-Acylamido-1-aryl-pyrazole sind durch die Formel (V) allgemein definiert. In dieser Formel (V) stehen $R^1$, $R^2$, X, A und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden. $E^3$ steht vorzugsweise für Chlor oder Brom.

Die 5-Acylamido-1-aryl-pyrazole der Formel (V) sind bekannt (vgl. US-PS 4 363 804 oder DE-OS

3 402 308) oder sie sind Gegenstand der eigenen vorgängigen noch nicht veröffentlichten Patentanmeldung DE-P 3 520 330 vom 07.06.1985 und erhältlich in Analogie zu bekannten Verfahren (vgl. z.B. DE-OS 3 402 308; vgl. auch Herstellungsbeispiele), beispielsweise wenn man 5-Amino-1-aryl-pyrazole der Formel (II),

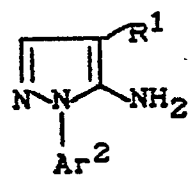

(II)

in welcher
$R^1$ und $Ar^2$ die oben angegebene Bedeutung haben,
mit Acylierungsmitteln der Formel (XII),

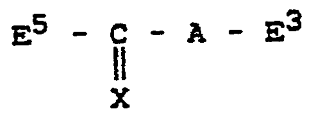

(XII)

in welcher
$E^3$, X und A die oben angegebene Bedeutung haben und
$E^5$ für eine Abgangsgruppe wie beispielsweise Halogen, insbesondere Chlor oder Brom, oder für einen Rest

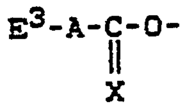

steht, wobei $E^3$, A und X die oben angegebene Bedeutung haben,
gegebenenfalls in Gegenwart eines Verdünnungsmittels, wie beispielsweise Dichlormethan, und gegebenenfalls in Gegenwart eines Säurebindemittels, wie beispielsweise Pyridin, bei Temperaturen zwischen –20°C und +120°C acyliert, und gegebenenfalls anschließend in Analogie zu bekannten Verfahren und in Analogie zu dem erfindungsgemäßen Verfahren (b) in einer 2. Stufe mit Alkylierungsmitteln der Formel (IV),
$E^2 - R^{2-1}$ (IV)
in welcher
$E^2$ und $R^{2-1}$ die oben angegebene Bedeutung haben,
gegebenenfalls in Gegenwart eines Verdünnungsmittels, wie beispielsweise Acetonitril, und gegebenenfalls in Gegenwart eines Katalysators wie beispielsweise Natriummethylat bei Temperaturen zwischen 0°C und 80°C alkyliert.

Die Acylierungsmittel der Formel (XII) sind allgemein bekannte Verbindungen der organischen Chemie.

Die zur Durchführung des erfindungsgemäßen Verfahrens (c) weiterhin als Ausgangsstoffe benötigten Phenol- bzw. Thiophenolderivate sind durch die Formel (VI) allgemein definiert. in dieser Formel (VI) stehen $Ar^1$ und Y vorzugsweise für diejenigen Reste, die bereits im Zusammenhang mit der Beschreibung der erfindungsgemäßen Stoffe der Formel (I) als bevorzugt für diese Substituenten genannt wurden, Z steht vorzugsweise für Wasserstoff oder für ein Natrium- oder Kaliumkation.

Die Phenol- bzw. Thiophenolderivate der Formel (VI) sind allgemein bekannte Verbindungen der organischen Chemie.

Die zur Durchführung des erfindungsgemäßen Verfahrens (d) als Ausgangsstoffe benötigten 5-Acylamido-1-aryl-pyrazole sind durch die Formel (Id) allgemein definiert, In dieser Formel (Id) stehen $R^1$, $R^2$, X, A, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Acylamido-1-aryl-pyrazole der Formel (Id) sind erfindungsgemäße Verbindungen und erhältlich mit Hilfe der erfindungsgemäßen Verfahren (a), (b), (c) oder (e).

Die zur Durchführung des erfindungsgemäßen Verfahrens (d) weiterhin als Ausgangsstoffe benötigten Oxidationsmittel sind durch die Formel (VII) allgemein definiert. In dieser Formel (VII) steht $R^3$ vorzugsweise für Wasserstoff, für Acetyl, Trifluoracetyl oder für gegebenenfalls substituiertes Benzoyl, wie beispielsweise 3-Chlorbenzoyl oder 4-Nitrobenzoyl.

Die Oxidationsmittel der Formel (VII) sind allgemein bekannte Verbindungen der organischen Chemie.

Die zur Durchführung des erfindungsgemäßen Verfahrens (e) als Ausgangsstoffe benötigten 5-Acylamido-i-aryl-pyrazole sind durch die Formel (If) allgemein definiert. In dieser Formel (If) stehen $R^2$, X, Y, A, $Ar^1$ und $Ar^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 5-Acylamido-1-aryl-pyrazole der Formel (If) sind erfindungsgemäße Verbindungen und erhältlich mit Hilfe der erfindungsgemäßen Verfahren (a), (b), (c) oder (d).

Die zur Durchführung des erfindungsgemäßen Verfahrens (e) weiterhin als Ausgangsstoffe benötigten Halogenierungs- oder Nitrierungsmittel sind durch die Formel (VIII) allgemein definiert. In dieser Formel (VIII) steht $R^1$ vorzugweise für Chlor, Brom oder Nitro.

$E^4$ steht vorzugsweise für eine übliche Abgangsgruppe, wie beispielsweise Halogen sowie phosphor- oder schwefelhaltige halogenierte Abgangsgruppen. Geeignete Halogenierungs- und Nitrierungsmittel sind beispielsweise Salpetersäure, Nitriersäure, Sulfurylchlorid, Phosphoroxychlorid, Phosphoroxybromid, Phosphortribromid und ähnliche allgemein übliche Halogenierungs- und Nitrierungsmittel.

Die Halogenierungs- und Nitrierungsmittel der Formel (VIII) sind allgemein bekannte Verbindungen.

Als Verdünnungsmittel zur Durchführung des erfindungsgemäßen Verfahrens (a) kommen inerte organische Lösungsmittel in Frage.

Hierzu gehören insbesondere aliphatische oder aromatische, gegebenenfalls, halogenierte Kohlenwasserstoffe, wie beispielsweise Benzin, Benzol Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid.

Das erfindungsgemäße Verfahren wird gegebenenfalls in Gegenwart eines geeigneten Säurebindemittels durchgeführt.

Als solche kommen alle üblichen anorganischen oder organischen Basen in Frage, Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Das erfindungsgemäßen Verfahren (a) kann auch gegebenenfalls in Gegenwart eines geeigneten Acylierungskatalysators durchgeführt werden. Als solche verwendet man vorzugsweise Protonensäure wie Schwefelsäure, Salzsäure, Phosphorsäure, Trifluoressigsäure oder Lewis-Säuren wie Aluminiumtrichlorid, Bortrifluorid oder Eisentrichlorid.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und +150 °C, vorzugsweise bei Temperaturen zwischen 0 °C und 100 °C.

Zur Durchführung des erfindungsgemäßen Verfahrens (a) setzt man pro Mol an 5-Amino-1-aryl-pyrazol der Formel (II) im allgemeinen 1.0 bis 15.0 Mol, vorzugsweise 1.0 bis 5.0 Mol an Acylierungsmittel der Formel (III), gegebenenfalls 1.0 bis 3.0 Mol, vorzugsweise 1.0 bis 2.0 Mol an Säurebindemittel oder gegebenenfalls 0.1 bis 3.0 Mol, vorzugsweise 0,1 bis 2.0 Mol an Acylierungskatalysator ein. Die Reaktionsdurchführung, Aufarbeitung und Isolierung der Reaktionsprodukte der Formel (Ia) erfolgt nach üblichen, bekannten Methoden.

Als Verdünnungsmittel zur Durchführung des erfindungsgemäßen Verfahrens (b) kommen ebenfalls inerte organische Lösungsmittel in Frage. Vorzugsweise verwendet man die bei Verfahren (a) genannten organischen Lösungsmittel.

Das erfindungsgemäße Verfahren (b) kann gegebenenfalls auch in einem Zweiphasensystem, wie beispielsweise Wasser/Toluol oder Wasser/Dichlormethan, gegebenenfalls in Gegenwart eines Phasentransferkatalysators, durchgeführt werden. Als Beispiele für solche Katalysatoren seien genannt: Tetrabutylammoniumiodid, Tetrabutylammoniumbromid, Tributyl-methylphosphoniumbromid, Trimethyl-$C_{13}/C_{15}$-alkylammoniumchlorid, Dibenzyl-ammoniummethylsulfat, Dimethyl-$C_{12}/C_{14}$-alkyl-benzylammoniumchlorid, Tetrabutylammoniumhydroxid, 15-Krone-5, 18-Krone-6, Triethylbenzylammoniumchlorid, Trimethylbenzylammoniumchlorid.

Als Säurebindemittel zur Durchführung des Herstellungsverfahrens (b) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen -20 °C und +150 °C, vorzugsweise bei Temperaturen zwischen 0 °C und +100 °C.

Zur Durchführung des erfindungsgemäßen Verfahrens (b) setzt man pro Mol an 5-Acylamido-1-arylpyrazol der Formel (Ia) im allgemeinen 1.0 bis 20.0 Mol, vorzugsweise 1.0 bis 15.0 Mol an Alkylierungsmittel der Formel (IV) und gegebenenfalls 1.0 bis 3.0 Mol, vorzugsweise 1.0 bis 2.0 Mol an Säurebindemittel sowie 0.01 bis 1.0 Mol an Phasentransferkatalysator ein. Die Reaktionsdurchführung, Aufarbeitung und Isolierung der Reaktionsprodukte der Formel (Ib) erfolgt in allgemein üblicher Art und Weise.

Als Verdünnungsmittel zur Durchführung des erfindungsgemäßen Verfahrens (c) kommen inerte organische Lösungsmittel oder wässrige Systeme in Frage. Vorzugsweise verwendet man die bei Verfahren (a) genannten organischen Lösungsmittel. Darüberhinaus bevorzugt sind polare organische Lösungsmittel wie Methanol, Ethanol, n-Propanol, i-Propanol, n-Butanol, i-Butanol, t-Butanol, Ethylenglykol, Diethylenglykol, Ethylenglykolmonomethylether oder -monoethylether, Diethylenglykolmonomethylether oder -monoethylether, deren Gemische mit Wasser oder auch reines Wasser als Verdünnungsmittel.

Das erfindungsgemäße Verfahren (c) wird gegebenenfalls in Gegenwart eines geeigneten Säurebindemittels durchgeführt. Als solche kommen alle üblicherweise verwendbaren anorganischen oder organischen Basen infrage. Vorzugsweise verwendet man die bei Verfahren (b) aufgezählten Basen als Säurebindemittel.

Das erfindungsgemäße Verfahren (c) kann gegebenenfalls auch in Gegenwart eines sauren Katalysators durchgeführt werden. Vorzugsweise verwendet man organische Protonensäuren wie Essigsäure oder Propionsäure oder starke Mineralsäuren wie Salzsäure, Schwefelsäure oder Phosphorsäure.

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +120 °C, vorzugsweise bei Temperaturen zwischen 0 °C und +100 °C.

Zur Durchführung des erfindungsgemäßen Verfahrens (c) setzt man pro Mol an 5-Acylamido-1-arylpyrazol der Formel (V) im allgemeinen 1.0 bis 5.0 Mol, vorzugsweise 1.0 bis 3.0 Mol an Phenol- bzw. Thiophenolderivat der Formel (VI) und gegebenenfalls 1.0 bis 5.0 Mol, vorzugsweise 1.0 bis 3.0 Mol an Säurebindemittel oder 0.001 bis 2.0 Mol, vorzugsweise 0.01 bis 1.2 Mol an saurem Katalysator ein. Die Reaktionsdurchführung, Aufarbeitung und Isolierung der Reaktionsprodukte der Formel (I) erfolgt in Analogie zu allgemein bekannten Verfahren.

Als Verdünnungsmittel zur Durchführung des erfindungsgemäßen Verfahrens (d) kommen ebenfalls inerte organische Lösungsmittel in Frage.

Vorzugsweise verwendet man Kohlenwasserstoffe, wie Benzin, Benzol, Toluol, Hexan oder Petrolether; chlorierte Kohlenwasserstoffe, wie Dichlormethan, 1,2-Dichlorethan, Chloroform, Tetrachlorkohlenstoff oder Chlorbenzol; Ether, wie Diethylether, Dioxan oder Tetrahydrofuran; Carbonsäuren, wie Essigsäure oder Propionsäure, oder dipolar aprotische Lösungsmittel, wie Acetonitril, Aceton, Essigsäureethylester oder Dimethylformamid.

Das erfindungsgemäße Verfahren (d) kann gegebenenfalls in Gegenwart eines Säurebindemittels durchgeführt werden. Als solche kommen alle üblicherweise verwendbaren organischen und anorganischen Säurebindemittel in Frage. Vorzugsweise verwendet man Erdalkali- oder Alkalimetallhydroxide, -acetate oder -carbonate, wie beispielsweise Calciumhydroxid, Natriumhydroxid, Natriumacetat oder Natriumcarbonat.

Das erfindungsgemäße Verfahren (d) kann gegebenenfalls in Gegenwart eines geeigneten Katalysators durchgeführt werden. Als solche kommen alle üblicherweise für derartige Schwefeloxidationen üblichen Katalysatoren wie insbesondere Metallsalze in Frage. Beispielhaft genannt sei in diesem Zusammenhang Ammoniummolybdat.

Die Reaktionstemperaturen können bei der Durchführung des erfindungsgemäßen Verfahrens (d) in einem größeren Bereich variiert werden. Im allgemeinen arbeitet man bei Temperaturen zwischen -20 °C und +70 °C, vorzugsweise bei Temperaturen zwischen 0°C und +50 °C.

Zur Durchführung des erfindungsgemäßen Verfahrens (d) setzt man pro Mol 5-Acylamido-1-aryl-pyrazol der Formel (Id) im allgemeinen 0.8 bis 1.2 Mol, vorzugsweise äquimolare Mengen Oxidationsmittel der Formel (VII) ein, wenn man die Oxidation des Schwefels auf der Sulfoxidstufe unterbrechen will. Zur Oxidation zum Sulfon setzt man pro Mol an 5-Acylamido-1-aryl-pyrazol der Formel (Id) im allgemeinen 1.8 bis 3.0 Mol, vorzugsweise doppelt molare Mengen an Oxidationsmittel der Formel (VII) ein. Die Reaktionsführung, Aufarbeitung und Isolierung der Endprodukte der Formel (Ic) erfolgt nach üblichen und bekannten Verfahren.

Als Verdünnungsmittel zur Durchführung des Herstellungsverfahrens (e) kommen alle üblicherweise für derartige elektrophile Substitutionen verwendbaren Lösungsmittel infrage, Vorzugsweise verwendet man die als Reagenzien in Frage kommenden Säuren oder Gemische, wie beispielsweise Salpetersäure, Nitriersäure oder Sulfurylchlorid gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel in Frage.

Als Katalysatoren oder Reaktionshilfsmittel zur Durchführung des Herstellungsverfahrens (e) kommen ebenfalls die für derartige Reaktionen üblichen Katalysatoren in Frage; vorzugsweise verwendet man saure Katalysatoren wie beispielsweise Schwefelsäure, Eisen-III-chlorid oder andere Lewis-Säuren oder Acetanhydrid.

Die Reaktionstemperaturen können bei der Durchführung des Herstellungsverfahrens (e) in einem größeren Bereich variiert werden. Im allgemeinen arbeitet man zwischen -50 °C und +200 °C, vorzugsweise zwischen -20 °C und +150 °C.

Zur Durchführung des Herstellungsverfahrens (e) setzt man pro Mol 5-Acylamido-1-aryl-pyrazol der Formel (If) im allgemeinen 1.0 bis 10.0 Mol, vorzugsweise 1.0 bis 5.0 Mol an Halogenierungs- oder Nitrierungsmittel der Formel (VIII) und gegebenenfalls 0.1 bis 10 Mol an Katalysator oder Reaktionshilfsmittel ein. Die Reaktionsführung, Aufarbeitung und Isolierung der Reaktionsprodukte der Formel (Ie) erfolgt in allgemein üblicher Art und Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in mono- und dikotylen Kulturen, wie beispielsweise Soja, Gerste, Weizen oder Mais einsetzen.

Auch die Zwischenprodukte der Formel (V) besitzen eine gute herbizide Wirksamkeit.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B., 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung im Getreide; 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch Mischungen mit N,N-Dimethyl-N'-(3-chlor-4-methylphenyl)-harnstoff; N,N-Dimethyl-N'-(4-isopropylphenyl)-harnstoff; 4-Amino-6-t-butyl-3-ethylthio-1,2,4-triazin-5(4H)-on; 2,4-Dichlorphenoxyessigsäure; 2,4-Dichlorphenoxypropionsäure; (2-Methyl-4-chlorphenoxy)-essigsäure; (4-Chlor-2-methylphenoxy)-propionsäure; Chloressigsäure-N-(methoxymethyl)-2,6-diethylanilid; 2-Ethyl-6-methyl-N-(1-methyl-2-methoxyethyl)-chloracetanilid; 2,6-Dinitro-4-trifluormethyl-N,N-dipropylanilin; 2-[4-(3,5-Dichlorpyrid-2-yloxy)-phenoxy]-propionsäure-(2-benzyloxyethylester);-(trimethylsilylmethylester) oder -(2,2-diethoxyethylester); 3,5-Diiod-4-hydroxybenzonitril); 3-Isopropyl-2,1,3-benzothiadiazin-4-on-2,2-dioxid; 2-Chlor-N-{[(4-methoxy-6-methyl-1,3,5-triazin-2-yl)-amino]-carbonyl}-benzolsulfonamid; N-(1-Ethylpropyl)-3,4-dimethyl-2,6-dinitroanilin; O-(6-chlor-3-phenyl-pyridazin-4-yl)-S-octyl-thiocarbonat; N,N-Diisopropyl-S-(2,3,3-trichlorallyl)-thiolcarbamat; N-Methyl-2-(1,3-benzthiazol-2-yloxy)-acetanilid; exo-1-Methyl-4-(1-methylethyl)-2-(2-methylphenyl-methoxy)-7-oxabicyclo-(2,2,1)-heptan; 2-{-4-[(3-Chlor-5-(trifluormethyl)-2-pyridinyl)-oxy]-phenoxy}-propionsäure bzw. -propansäureethylester;3,5-Dibrom-4-hydroxy-benzonitril; 2-[5-Methyl-5-(1-methylethyl)-4-oxo-2-imidazolin-2-yl]-3-chinolincarbonsäure oder 1-(3-Trifluormethyl-phenyl)-4-methylamino-5-chlor-pyridazon-(6) sind gegebenenfalls von Vorteil.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

<u>Herstellungsbeispiele:</u>

<u>Beispiel 1</u>:

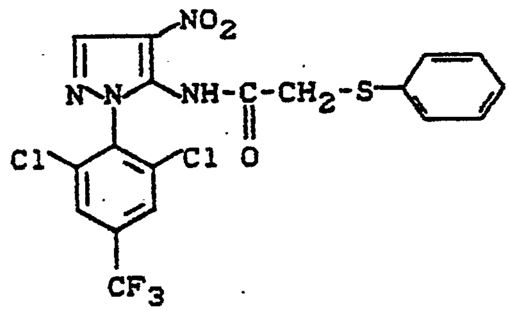

(Verfahren c)

Zu 0,85 g (0,0283 Mol) 80-prozentigem Natriumhydrid in 50 ml trockenem Dimethoxyethan gibt man unter Stickstoff bei 15°C–20°C nacheinander 3,1g (0,0282 Mol) Thiophenol und 10g (0.024 Mol) 5-Chloracetamido-4-nitro-1-(2,6-dichlor-4-trifluormethyl-phenyl)-pyrazol gelöst in 50ml trockenem Dimethoxyethan. Nach 3-stündigem Rühren tropft man 25 ml Ethanol zu und engt im Vakuum ein. Der ölige Rückstand wird chromatographisch (Kieselgel; Laufmittel: Dichlormethan) gereinigt. Man erhält 9,5 g (81% der Theorie) 4-Nitro-5-(phenylthio-acetamido)-1-(2,6-dichlor-4-trifluormethyl-phenyl)-pyrazol vom Schmelzpunkt 98°C–99°C.

<u>Beispiel 2:</u>

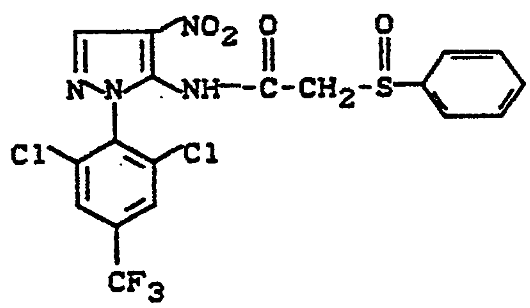

(Verfahren d)

3,0 g (0.0061 Mol) 4-Nitro-5-phenylthioacetamido-1-(2,6-dichlor-4-trifluormethyl-phenyl)-pyrazol und 1,4 g (0.0073 Mol) 90-prozentige 3-Chlorperbenzoesäure in 30 ml Dichlormethan werden 16 Stunden bei Raumtemperatur gerührt, filtriert, das Filtrat nacheinander mit Natriumbicarbonat, Natriumthiosulfat-, nochmals mit Natriumbicarbonat- und dann mit Natriumchloridlösung gewaschen, über Magnesiumsulfat getrocknet und im Vakuum vom Lösungsmittel befreit. Man erhält 3,0 g (96,8% der Theorie) an 4-Nitro-5-phenylsulfinylacetamido-1-(2,6-dichlor-4-trifluormethyl-phenyl)-pyrazol vom Schmelzpunkt 108°C–110°C.

<u>Beispiel 3:</u>

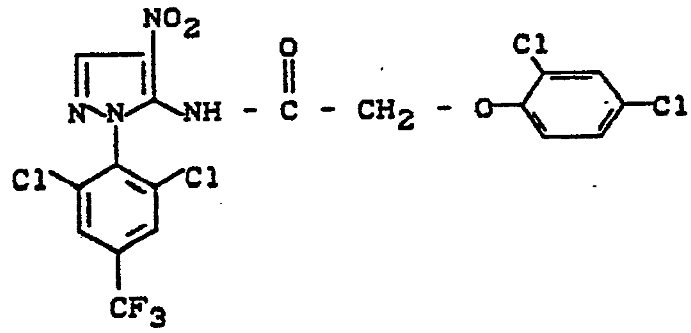

(Verfahren e)

Zu 7,0 g (0,014 Mol) 5-(2,4-Dichlorphenoxyacetamido)-1-(2,6-dichlor-4-trirluormethyl-phenyl)-pyrazol in 30 ml Eisessig gibt man bei ca. 150°C nacheinander 1,45 ml (0,0154 Mol) Acetanhydrid und 0,60 ml (0,014 Mol) 98prozentige Salpetersäure. Nach 20stündigem Rühren wird die Mischung im Vakuum eingeengt, der Rückstand in 100 ml Dichlormethan aufgenommen und nacheinander mit gesättigter Natriumhydrogencarbonat- und gesättigter Kochsalzlösung gewaschen. Die organische Phase wird über Magnesiumsulfat getrocknet und im Vakuum vom Lösungsmittel befreit. Man erhält 8,6 g (81,6% der Theorie) an 5-(2,4-Dichlorphenoxyacetamido)-4-nitro-1-(2,6-dichlor-4-trifluormethylphenyl)-pyrazol vom Schmelzpunkt 155°C–160°C.

Beispiel 4:

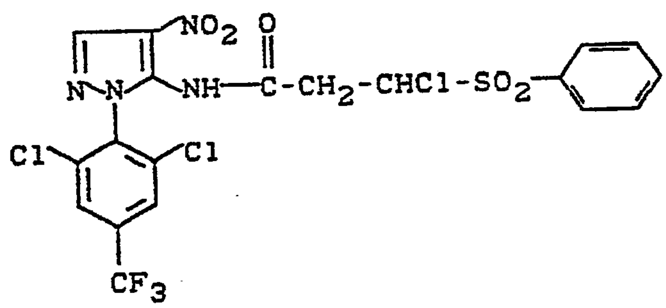

(Verfahren a)

Zu 4 g (0,117 Mol) 5-Amino-4-nitro-1-(2,6-dichlor-4-trifluormethyl-phenyl)-pyrazol in 30 ml 1,2-Dichlorbenzol gibt man 6,6 g (0,247 Mol) 3-Chlor-3-phenylsulfonylpropionsäurechlorid und zwei Tropfen 96prozentige Schwefelsäure und erhitzt 6 Stunden auf 100°C. Das Reaktionsgemisch wird im Vakuum vom Lösungsmittel befreit und säulenchromatographisch mit Kieselgel als Trägermaterial und Dichlormethan als Laufmittel getrennt. Man erhält 1,7 g (25,4% der Theorie) 5-(3-Chlor-3-phenylsulfonylpropionylamido)-4-nitro-1-(2, 6-dichlor-4-trifluormethylphenyl)-pyrazol vom Schmelzpunkt 122°C.

In entsprechender Weise und gemäß den allgemeinen Angaben zur Herstellung erhält man die folgenden 5-Acylamido-1aryl-pyrazole der allgemeinen Formel (I):

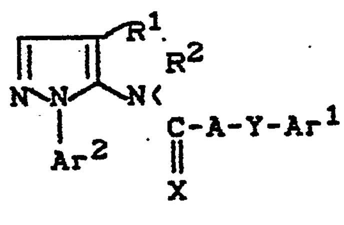

(I)

<u>Tabelle 2</u>

| Bsp. Nr. | $R^1$ | $R^2$ | $-\overset{X}{\overset{\|}{C}}-A-Y-Ar^1$ | $Ar^2$ | Schmelzpunkt/°C |
|---|---|---|---|---|---|
| 5 | $NO_2$ | H | 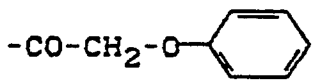 | 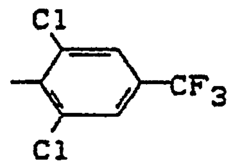 | 144-148 |
| 6 | $NO_2$ | H | 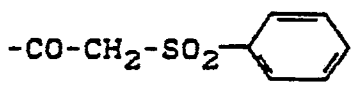 | 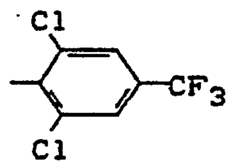 | 162-164 |
| 7 | $NO_2$ | H | 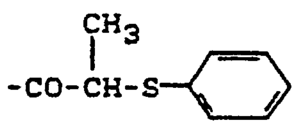 | 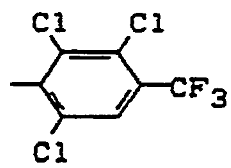 | |
| 8 | $NO_2$ | H | 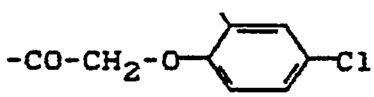 | 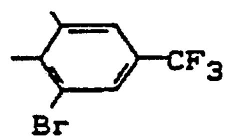 | 156-160 |
| 9 | $NO_2$ | H | 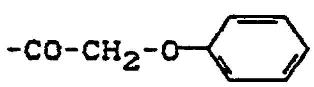 | 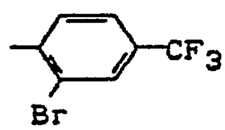 | 80-90 |
| 10 | $NO_2$ | H | 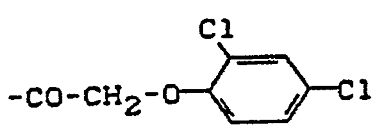 | 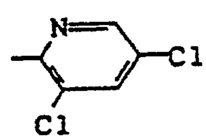 | 165 |
| 11 | $NO_2$ | H | 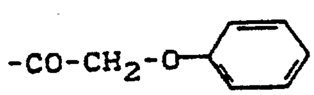 | 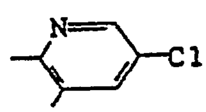 | 142(Zers. |

Tabelle 2 (Fortsetzung)

| Bsp. Nr. | $R^1$ | $R^2$ | $-\overset{X}{\overset{\Vert}{C}}-A-Y-Ar^1$ | $Ar^2$ | Schmelzpunkt/°C |
|---|---|---|---|---|---|
| 12 | $NO_2$ | H | $-CO-CH_2-O-$ 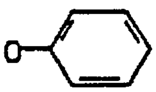 | 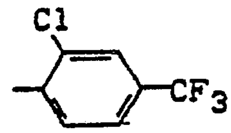 | 177-180 |
| 13 | $NO_2$ | H | $-CO-CH_2-O-$ 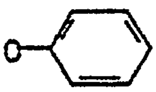 | 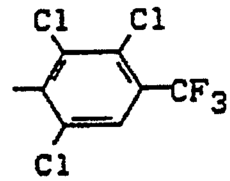 | 110-113 |
| 14 | $NO_2$ | H | $-CO-CH_2-O-$ 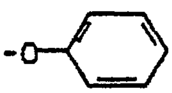 | 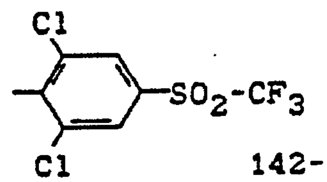 | 142-144 |
| 15 | $NO_2$ | H | $-CO-CH_2-O-$ 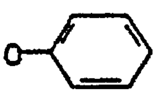 | 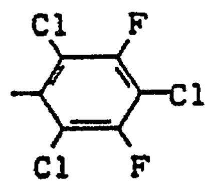 | 183-186 |

Tabelle 2 (Fortsetzung)

| Bsp. Nr. | $R^1$ | $R^2$ | 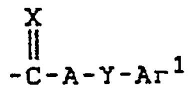 | $Ar^2$ | Schmelzpunkt/°C |
|---|---|---|---|---|---|
| 16 | $NO_2$ | H | 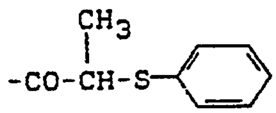 | 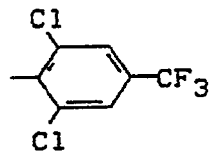 | 106-109 |
| 17 | $NO_2$ | H | 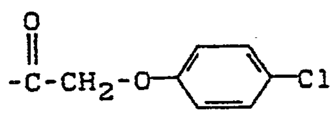 | 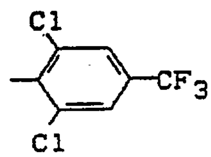 | 141-145 |
| 18 | $NO_2$ | H | 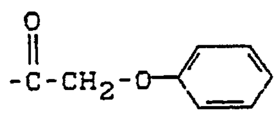 | 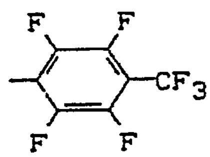 | 112-113 |
| 19 | $NO_2$ | H | 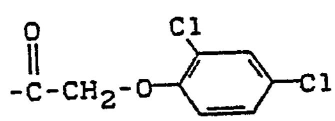 | 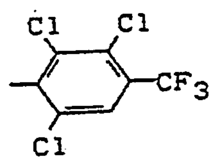 | 162-165 |
| 20 | $NO_2$ | H | 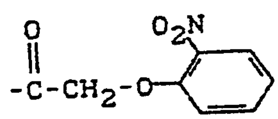 | 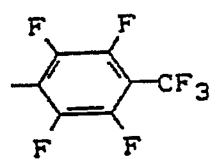 | 158-169 |
| 21 | $NO_2$ | H | 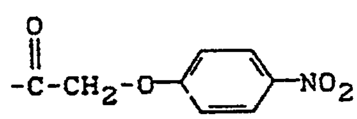 | 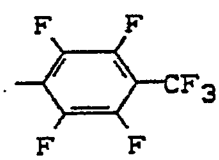 | 158-169 |
| 22 | $NO_2$ | H | 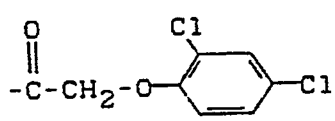 | 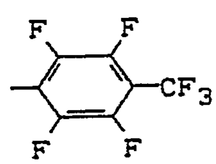 | 152-157 |
| 23 | $NO_2$ | H | 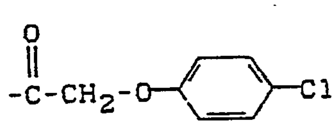 | 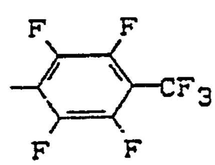 | 48-54 |

Herstellung der Ausgangsverbindungen:

Beispiel V-1:

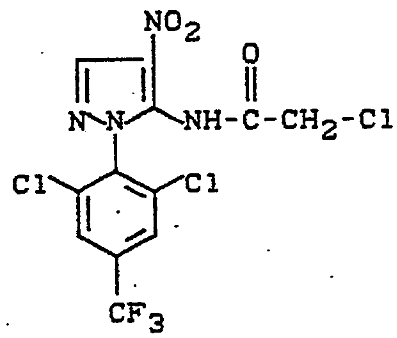

Zu 4,2 (0,011 Mol) 5-Chloracetamido-1-(2,6-dichlor-4-trifluormethyl-phenyl)-pyrazol in 20 ml Eisessig gibt man bei 15°C nacheinander 1,03 ml (0,011 Mol) Acetanhydrid und 0,56 ml (0,013 Mol) 98prozentige Salpetersäure. Nach 6stündigem Rühren wird die Mischung im Vakuum eingeengt, der Rückstand in 100 ml Dichlormethan aufgenommen und nacheinander mit gesättigter Natriumhydrocarbonat- und gesättigter Kochsalzlösung gewaschen. Die organische Phase wird über Magnesiumsulfat getrocknet und im Vakuum vom Lösungsmittel befreit. Man erhält 4,1 g (89% beider Theorie) an 5-Chloracetamido-4-nitro-1-(2,6-dichlor-4-trifluormethyl-phenyl)-pyrazol vom Schmelzpunkt 130°C–133°C.

Analog erhält man

Beispiel V-2:

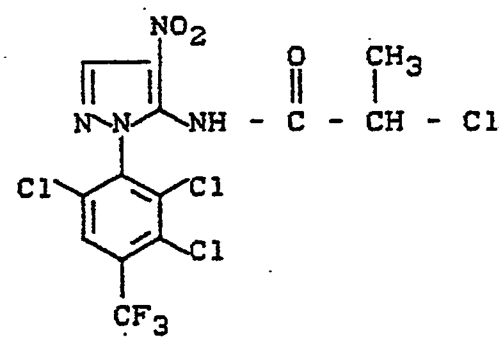

Schmelzpunkt 50°C–52°C.

Beispiel V-3:

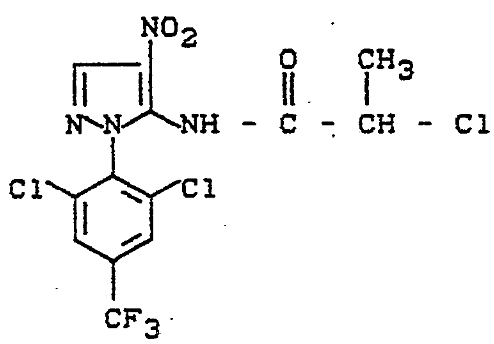

Schmelzpunkt: 53°C–56°C.

Anwendungsbeispiele:

In den folgenden Anwendungsbeispielen wurde die nachstehend aufgeführte Verbindung als Vergleichsubstanz eingesetzt:

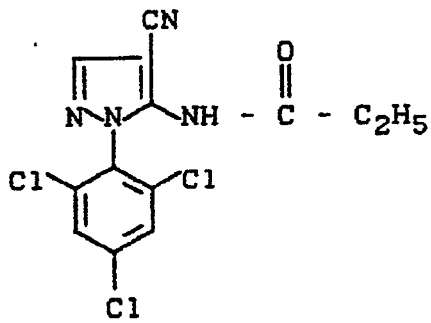

(A)

4-Cyano-5-propionamido-1-(2,4,6-trichlorphenyl)-pyrazol (bekannt aus DE-OS 3 226 513)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen: 4 und 7.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber der Vergleichssubstanz (A) zeigen in diesem Test z.B. die Verbindungen gemäß den Herstellungsbeispielen: 2, 4, 7 und 10.

**Patentansprüche**

1. 5-Acylamido-1-aryl-pyrazole der allgemeinen Formel (I),

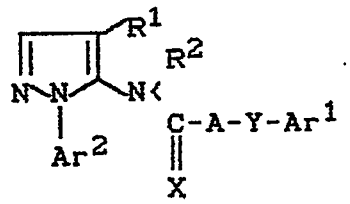

(I)

in welcher
$R^1$ für Nitro, Fluor, Chlor, Brom oder Jod steht,
$R^2$ für Wasserstoff oder für geradkettiges oder verzweigtes Alkyl mit 1 bis 12 Kohlenstoffatomen steht,
X für Sauerstoff oder Schwefel steht,
Y für Sauerstoff, Schwefel, eine Sulfinyl- oder eine Sulfonylgruppe steht,
A für eine geradkettige oder verzweigte, gegebenenfalls einfach oder mehrfach, gleich oder verschieden durch Halogen substituierte Alkylenbrücke mit 1 bis 6 Kohlenstoffatomen steht,
$Ar^1$ für jeweils gegebenenfalls einfach oder mehrfach, gleich oder verschieden substituiertes Phenyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, Pyrazinyl oder s-Triazinyl steht, wobei als Substituenten jeweils in Frage kommen: Halogen, Cyano, Nitro, jeweils geradkettiges oder verzweigtes Alkyl, Alkoxy oder Alkoxycarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$, wobei
$R^4$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und
m für eine Zahl 0, 1 oder 2 steht sowie
$Ar^2$ für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils die bei $Ar^1$ genannten in Frage kommen.

2. 5-Acylamido-1-aryl-pyrazole der Formel (I) gemäß Anspruch 1, bei welchen
$R^1$ für Nitro, Chlor oder Brom steht,
$R^2$ für Wasserstoff oder für geradkettiges oder verzweigtes Alkyl mit 1 bis 8 Kohlenstoffatomen steht,
X für Sauerstoff oder Schwefel steht,
Y für Sauerstoff, Schwefel, eine Sulfinylgruppe oder eine Sulfonylgruppe steht,
A für ein Brückenglied der Formel $-CH_2-$; $-CH_2-CH_2-$;

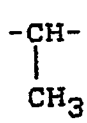 ; $-CCl_2-CH_2-$ ; $-CHCl-CH_2-$ ; 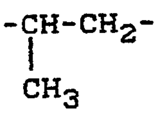 ; $-CH_2-CH_2-CH_2-$ ;

$-CH_2-CH_2-CH_2-CH_2-$ ; 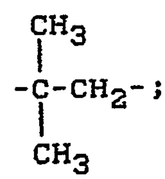 ; 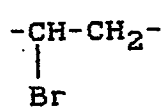 oder $-CBr_2-CH_2-$

steht,
$Ar^1$ für jeweils gegebenenfalls ein- bis dreifach, gleich oder verschieden substituiertes Phenyl, 2-Pyridyl, 3-Pyridyl, 4-Pyridyl, 2-Pyrimidyl, 4-Pyrimidyl, 5-Pyrimidyl, 3-Pyridazinyl, 4-Pyridazinyl, Pyrazinyl oder s-Triazinyl steht, wobei als Substituenten jeweils in Frage kommen: Cyano, Nitro, Fluor, Chlor, Brom, Iod, Methyl, Ethyl, n- oder i-Propyl, n-, i-, s- oder t-Butyl, Methoxy, Ethoxy, Methoxycarbonyl, Ethoxycarbonyl, Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifuordichlorethoxy, Pentachlorethoxy oder ein Rest $-S(O)_m-R^4$, wobei
$R^4$ für Amino, Methylamino, Ethylamino, Dimethylamino, Diethylamino, Fluordichlormethyl, Difluormethyl, Tetrafluorethyl, Trichlorethyl, Trifluormethyl, Difluorchlormethyl, Methyl oder Ethyl steht und
m für eine Zahl 0, 1 oder 2 steht sowie

$Ar^2$ für gegebenfalls ein- bis fünffach, gleich oder verschieden substituiertes Phenyl oder für jeweils gegebenfalls ein- bis vierfach, gleich oder verschieden substituiertes 2-Pyridyl oder 4-Pyridyl steht, wobei als Substituenten jeweils die bei $Ar^1$ genannten in Frage kommen.

3. Verfahren zur Herstellung von 5-Acylamido-1-aryl-pyrazolen der Formel (I)

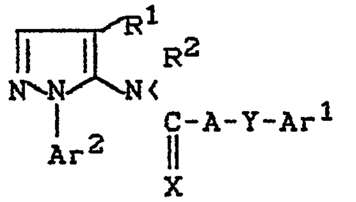

(I)

in welcher

$R^1$ für Nitro, Fluor, Chlor, Brom oder Iod steht,

$R^2$ für Wasserstoff oder für geradkettiges oder verzweigtes Alkyl mit 1 bis 12 Kohlenstoffatomen steht,

X für Sauerstoff oder Schwefel steht,

Y für Sauerstoff, Schwefel, eine Sulfinyl- oder eine Sulfonylgruppe steht,

A für eine geradkettige oder verzweigte, gegebenenfalls einfach oder mehrfach, gleich oder verschieden durch Halogen substituierte Alkylenbrücke mit 1 bis 6 Kohlenstoffatomen steht,

$Ar^1$ für jeweils gegebenfalls einfach oder mehrfach, gleich oder verschieden substituiertes Phenyl, 2-Pyridiyl, 3-Pyridyl, 4-Pyridyl, 2-Pyrimidyl, 5-Pyrimidyl, 3-Pyridazinyl, 4-Pyridazinyl, Pyrazinyl oder s-Triazinyl steht, wobei als Substituenten jeweils in Frage kommen: Halogen, Cyano, Nitro, jeweils geradkettiges oder verzweigtes Alkyl, Alkoxy oder Alkoxyxcarbonyl mit jeweils 1 bis 4 Kohlenstoffatomen in den Alkylteilen, jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder ein Rest $-S(O)_m-R^4$, wobei

$R^4$ für Amino, für jeweils geradkettiges oder verzweigstes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

m für eine Zahl 0, 1 oder 2 steht sowie

$Ar^2$ für jeweils gegebenenfalls einfach oder mehrfach, gleich oder verschieden substituiertes Phenyl, 2-Pyridyl, 3-Pyridyl oder 4-Pyridyl steht, wobei als Substituenten jeweils die bei $Ar^1$ genannten in Frage kommen.

dadurch gekennzeichnet, daß man

a) 5-Alcylamido-1-aryl-pyrazole der Formel (Ia),

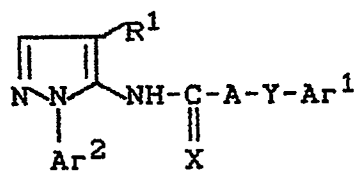

(Ia)

in welcher

$R^1$, X, Y, A, $Ar^1$ und $Ar^2$ die oben angegebene Bedeutung haben,

erhält, wenn man 5-Amino-1-aryl-pyrazole der Formel (II),

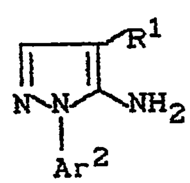

(II)

in welcher

$R^1$ und $Ar^2$ die oben angegebene Bedeutung haben, mit Acylierungsmitteln der Formel (III),

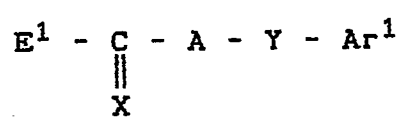

in welcher
X, Y, A und $Ar^1$ die oben angegebene Bedeutung haben und
$E^1$ für eine elektronenanziehende Abgangsgruppe steht,
gegebenenfalls in Gegenwart eines Verdünnungsmittels, gegebenenfalls in Gegenwart eines Säurebindemittels sowie gegebenenfalls in Gegenwart eines Katalysators umsetzt;
oder daß man
b) 5-Acylamido-1-aryl-pyrazole der Formel (Ib),

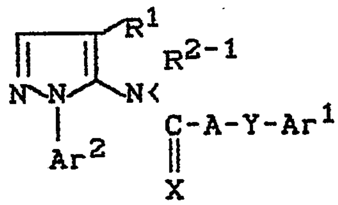
(Ib)

in welcher
$R^1$, X, Y, A, $Ar^1$ und $Ar^2$ die angegebene Bedeutung haben und
$R^{2-1}$ für geradkettiges oder verzweigtes Alkyl mit 1 bis 12 Kohlenstoffatomen steht,
erhält, wenn man die nach Verfahren (a) erhältlichen 5-Acylamido-1-aryl-pyrazole der Formel (Ia),

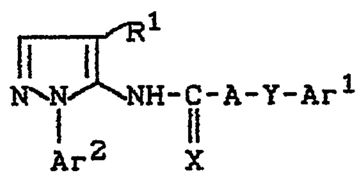
(Ia)

in welcher
$R^1$, X, Y, A, $Ar^1$ und $Ar^2$ die oben angegebene Bedeutung haben,
mit Alkylierungsmitteln der Formel (IV),
$E^2 - R^{2-1}$ (IV)
in welcher
$R^{2-1}$ die oben angegebene Bedeutung hat und
$E^2$ für Halogen, für gegebenenfalls substituiertes Alkoxysulfonyloxy oder für gegebenenfalls substituiertes Arylsulfonyloxy steht, gegebenenfalls in Gegenwart eines Verdünnungsmittels und gegebenenfalls in Gegenwart eines Säurebindemittels sowie gegebenenfalls in Gegenwart eines Katalysators umsetzt;
oder daß man
c) 5-Alcylamido-1-aryl-pyrazole der Formel (I) erhält, wenn man 5-Acylamido-1-aryl-pyrazole der Formel (V),

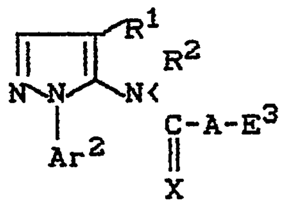
(V)

in welcher
$R^1$, $R^2$, X, A und $Ar^2$ die oben angegebene Bedeutung haben und
$E^3$ für Halogen steht
mit Phenol- bzw. Thiophenolderivaten der Formel (VI),
$Ar^1 - Y - Z$ (VI)
in welcher
$Ar^1$ und Y die oben angegebene Bedeutung haben und
Z für Wasserstoff oder für ein Äquivalent eines Erdakali- oder Alaklimetallkations steht, gegebenenfalls in Gegenwart eines Verdünnungsmittels und gegebenenfalls in Gegenwart eines Säurebindemittels und gegebenenfalls in Gegenwart eines geeigneten Katalysators umsetzt,
oder daß man
d) 5-Acylamido-1-aryl-pyrazole der Formel (Ic)

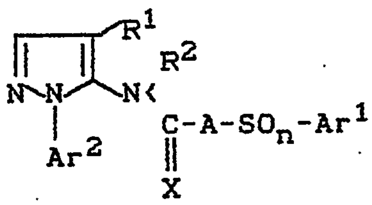

(Ic)

in welcher
$R^1$, $R^2$, X, A, $Ar^1$ und $Ar^2$ die oben angegebene Bedeutung haben und
n für eine Zahl 1 oder 2 steht,
erhält, wenn man die mit Hilfe der Verfahren (a), (b) oder (c) erhältlichen 5-Acylamido-1-aryl-pyrazole der Formel (Id),

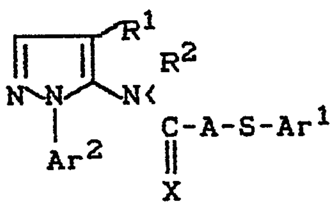

(Id)

in welcher
$R^1$, $R^2$, X, A, $Ar^1$ und $Ar^2$ dei oben angegebenen Bedeutung haben,
mit Oxidationsmittel der Fromel (VII),
$R^3-O-O-H$ (VII)
in welcher
$R^3$ für Wasserstoff oder für jeweils gegebenenfalls substituiertes Alkanoyl oder Aroyl steht,
gegebenenfalls in Gegenwart eines Verdünnungsmittels und gegebenenfalls in Gegenwart eines Säurebindemittels sowie gegebenenfalls in Gegenwart eines Katalysators umsetzt,
oder daß man
e) 5-Acylamido-1-aryl-pyrazole der Formel (Ie)

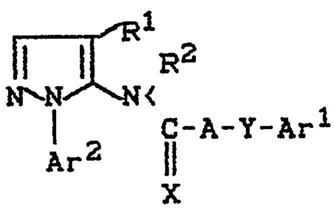

(Ie)

in welcher
$R^2$, X, A, Y, $Ar^1$ und $Ar^2$ die oben angegebene Bedeutung haben und
$R^1$ für Nitro, Fluor, Chlor, Brom oder Iod steht, erhält, wenn man die mit Hilfe der Verfahren (a), (b), (c) oder (d) erhältlichen 5-Acylamido-1-aryl-pyrazole der Formel (If),

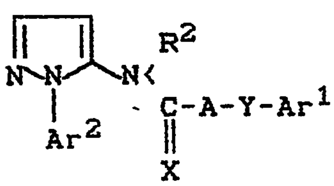

(If)

in welcher
$R^2$, X, Y, A, $Ar^1$ und $Ar^2$ die oben angegebene Bedeutung haben,
mit Halogenierungs- oder Nitrierungsmitteln der Formel (VIII),
$R^1-E^4$ (VIII)
in welcher
$R^1$ die oben angegebene Bedeutung hat und
$E^4$ für eine elektronenanziehende Abgangsgruppe steht, gegebenenfalls in Gegenwart eines Verdünnungsmittels und gegebenenfalls in Gegenwart eines Katalysators oder Reaktionshilfsmittels umsetzt.

4. Herbizide Mittel, gekennzeichnet durch einen Gehalt an mindestens einem 5-Acylamido-1aryl-pyrazol der Formel (I) gemäß den Ansprüchen 1 bis 3.

5. Verfahren zur Bekämpfung von Unkräutern, dadurch gekennzeichnet, daß man 5-Acylamido-1-aryl-pyrazole der Formel (I) gemäß den Ansprüchen 1 bis 3 auf die Unkräuter und/oder ihren Lebensraum einwirken läßt.

6. Verwendung von 5-Acylamido-1-aryl-pyrazolen der Formel (I) gemäß den Ansprüchen 1 bis 3 zur Bekämpfung von Unkräutern.

7. Verfahren zur Herstellung von herbiziden Mitteln, dadurch gekennzeichnet, daß man 5-Acylamido-1-aryl-pyrazole der Formel (I) gemäß den Ansprüchen 1 bis 3 mit Streckmitteln und/oder oberflächenaktiven Substanzen vermischt.

**Claims**

1. 5-Acylamido-1-aryl-pyrazoles of the general formula (I),

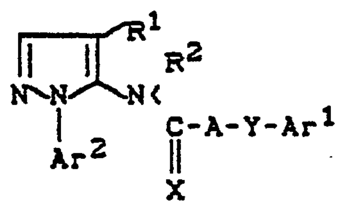

(I)

in which
$R^1$ represents nitro, fluorine, chlorine, bromine or iodine,
$R^2$ represents hydrogen or straight-chain or branched alkyl having 1 to 12 carbon atoms,
X represents oxygen or sulphur,
Y represents oxygen, sulphur, a sulphinyl or a sulphonyl group,
A represents a straight-chain or branched, optionally singly or multiply, identically or differently halo-substituted alkaline bridge having 1 to 6 carbon atoms,
$Ar^1$ represents in each case optionally singly or multiply, identically or differently substituted phenyl, 2-pyridyl, 3-pyridyl, 4-pyridyl, 2-pyrimidyl, 4-pyrimidyl, 5-pyrimidyl, 3-pyridazinyl, 4-pyridazinyl, pyrazinyl or s-triazinyl, suitable substituents in each case being: halogen, cyano, nitro in each case straight-chain or branched alkyl, alkoxy or alkoxycarbonyl having 1 to 4 carbon atoms in each of the alkyl parts, in each case straight-chain or branched haloalkyl or haloalkoxy having 1 to 4 carbon atoms and 1 to 9 identical or different halogen atoms in each case, or a $-S(O)_m-R^4$ radical,
where
$R^4$ represents amino, in each case straight-chain or branched alkyl, alkylamino, dialkylamino or haloalkyl having 1 to 4 carbon atoms in each of the individual alkyl parts and, in the case of haloalkyl, having 1 to 9 identical or different halogen atoms and
m represents a number 0, 1 or 2 and
$Ar^2$ represents in each case optionally singly or multiply, identically or differently, substituted phenyl, 2-pyridyl, 3-pyridyl or 4-pyridyl, suitable substituents in each case being those mentioned for $Ar^1$.

2. 5-Acylamido-1-aryl-pyrazoles of the formula (I), according to claim 1, in which
$R^1$ represents nitro, chlorine or bromine,
$R^2$ represents hydrogen or straight-chain or branched alkyl having 1 to 8 carbon atoms,
X represents oxygen or sulphur,
Y represents oxygen, sulphur, a sulphinyl group or a sulphonyl group,
A represents a bridging member of the formula

$$-CH_2-;\ -CH_2-CH_2-;\ -CH(CH_3)-;\ -CCl_2-CH_2-;\ -CHCl-CH_2-;$$

$$-CH(CH_3)-CH_2-;\ -CH_2-CH_2-CH_2-;\ -CH_2-CH_2-CH_2-CH_2-;\ -C(CH_3)_2-CH_2-;$$

$$-CH(Br)-CH_2-\ \text{or}\ -CBr_2-CH_2-,$$

$Ar^1$ represents in each case optionally singly to triply, identically or differently, substituted phenyl, 2-pyridyl, 3-pyridyl, 4-pyridyl, 2-pyrimidyl, 4-pyrimidyl, 5-pyrimidyl, 3-pyridazinyl, 4-pyridazinyl, pyrazinyl or s-triazinyl, suitable substituents in each case being: cyano, nitro, fluorine, chlorine, bromine, iodine, methyl, ethyl, n- or i-propyl, n-, i-, s- or t-butyl, methoxy, ethoxy, methoxycarbonyl, ethoxycarbonyl, trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl, pentachloroethyl, trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy, pentachloroethoxy or a $-S(O)_m-R^4$ radical,
where
$R^4$ represents amino, methylamino, ethylamino, dimethylamino, diethylamino, fluorodichloromethyl, difluoromethyl, tetrafluoroethyl, trichloroethyl, trifluoromethyl, difluorochloromethyl, methyl or ethyl and
m represents a number 0, 1 or 2 and
$Ar^2$ represents optionally singly to quintuply, identically or differently, substituted phenyl or in each case optionally singly to quadruply, identically or differently, substituted 2-pyridyl or 4-pyridyl, suitable substituents in each case being those mentioned for $Ar^1$.

3. Process for the preparation of 5-acylamido-1aryl-pyrazoles of the formula (I)

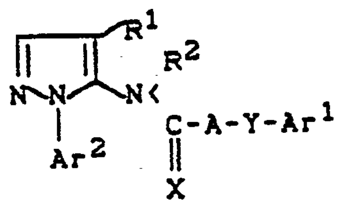

(I)

in which
$R^1$ represents nitro, fluorine, chlorine, bromine or iodine,
$R^2$ represents hydrogen or straight-chain or branched alkyl having 1 to 12 carbon atoms,
X represents oxygen or sulphur,
Y represents oxygen, sulphur, a sulphinyl or a sulphonyl group,
A represents a straight-chain or branched, optionally singly or multiply, identically or differently halo-substituted alkylene bridge having 1 to 6 carbon atoms,
$Ar^1$ represents in each case optionally singly or multiply, identically or differently substituted phenyl, 2-pyridyl, 3-pyridyl, 4-pyridyl, 2-pyrimidyl, 4-pyrimidyl, 5-pyrimidyl, 3-pyridazinyl, 4-pyridazinyl, pyrazinyl or s-triazinyl, suitable substituents in each case being: halogen, cyano, nitro, in each case straight-chain or branched alkyl, alkoxy or alkoxycarbonyl having 1 to 4 carbon atoms in each of the alkyl parts, in each case straight-chain or branched haloalkyl or haloalkoxy having 1 to 4 carbon atoms and 1 to 9 identical or different halogen atoms in each case, or a $-S(O)_m-R^4$ radical,
where
$R^4$ represents amino, in each case straight-chain or branched alkyl, alkylamino, dialkylamino or haloalkyl having 1 to 4 carbon atoms in each of the individual alkyl parts and, in the case of haloalkyl, having 1 to 9 identical or different halogen atoms and
m represents a number 0, 1 or 2 and
$Ar^2$ represents in each case optionally singly or multiply, identically or differently, substituted phenyl, 2-pyridyl, 3-pyridyl or 4-pyridyl, suitable substituents in each case being those mentioned for $Ar^1$,
characterized in that

a) 5-acylamido-1-aryl-pyrazoles of the formula (Ia),

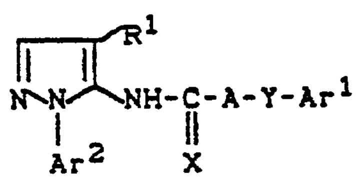

(Ia)

in which
$R^1$, X, Y, A, $Ar^1$ and $Ar^2$ have the abovementioned meaning, are obtained when 5-amino-1-aryl-pyrazoles of the formula (II)

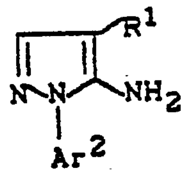

(II)

in which
$R^1$ and $Ar^2$ have the abovementioned meaning, are reacted with acylating agents of the formula (III),

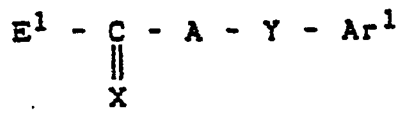

in which
X, Y, A and $Ar^1$ have the abovementioned meaning and
$E^1$ represents an electron-withdrawing leaving group, if appropriate in the presence of a diluent, if appropriate in the presence of an acid acceptor and if appropriate in the presence of a catalyst;
or in that,
b) 5-acylamido-1-aryl-pyrazoles of the formula (Ib)

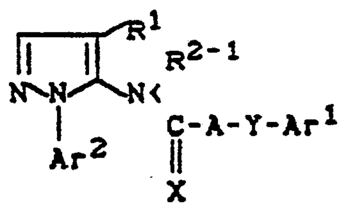

(Ib)

in which
$R^1$, X, Y, A, $Ar^1$ and $Ar^2$ have the abovementioned meaning and
$R^{2-1}$ represents straight-chain or branched alkyl having 1 to 12 carbon atoms,
are obtained when the 5-acylamido-1-aryl-pyrazoles of the formula (Ia),

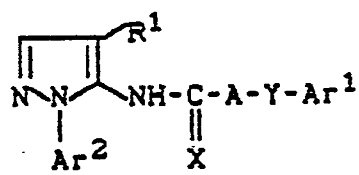

(Ia)

in which $R^1$, X, Y, A, $Ar^1$ and $Ar^2$ have the abovementioned meaning,
which are obtained by process (a) are reacted with alkylating agents of the formula (IV),
$E^2 - R^{2-1}$ (IV)
in which
$R^{2-1}$ has the abovementioned meaning and
$E^2$ represents halogen, optionally substituted alkoxysulphonyloxy or optionally substituted arylsulphonyloxy,
if appropriate in the presence of a diluent and if appropriate in the presence of an acid acceptor, and also if appropriate in the presence of a catalyst;
or in that
c) 5-acylamido-1-aryl-pyrazoles of the formula (I) are obtained when 5-acylamido-1-aryl-pyrazoles of the formula (V)

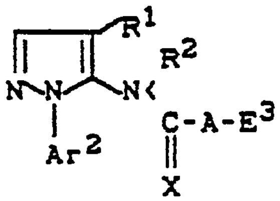

(V)

in which
$R^1$, $R^2$, X, A and $Ar^2$ have the abovementioned meaning and
$E^3$ represents halogen
are reacted with phenol or thiophenol derivatives of the formula (VI)

$Ar^1 - Y - Z$ (VI)

in which
$Ar^1$ and Y have the abovementioned meaning and
Z represents hydrogen or one equivalent of an alkaline earth metal cation or alkali metal cation,
if appropriate in the presence of a diluent and if appropriate in the presence of an acid acceptor and if appropriate in the presence of a suitable catalyst;
or in that
d) 5-acylamido-1-aryl-pyrazoles of the formula (Ic),

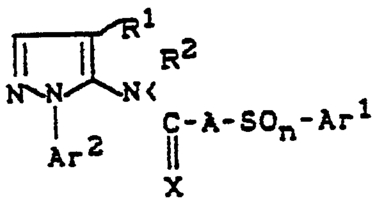

(Ic)

in which
$R^1$, $R^2$, X, A, $Ar^1$ and $Ar^2$ have the abovementioned meaning and
n represents a number 1 or 2,
are obtained when the 5-acylamido-1-aryl-pyrazoles of the formula (Id)

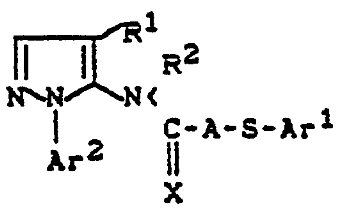

(Id)

in which
$R^1$, $R^2$, X, A, $Ar^1$ and $Ar^2$ have the abovementioned meaning,
which are obtained with the aid of the processes (a), (b) or (c), are reacted with oxidizing agents of the formula (VII),

$R^3 - O - O - H$ (VII)

in which
$R^3$ represents hydrogen or in each case optionally substituted alkanoyl or aroyl, if appropriate in the presence of a diluent and if appropriate in the presence of an acid acceptor, and also if appropriate in the presence of a catalyst;
or in that
e) 5-acylamido-1-aryl-pyrazoles of the formula (Ie),

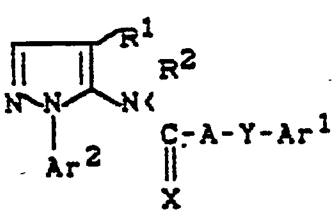

(Ie)

in which

$R^2$, X, A, Y, $Ar^1$ and $Ar^2$ have the abovementioned meaning and
$R^1$ represents nitro, fluorine, chlorine, bromine or iodine,
are obtained when the 5-acylamido-1-aryl-pyrazoles of the formula (If),

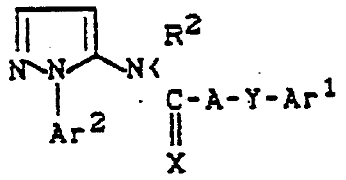

(If)

in which
$R^2$, X, Y, A, $Ar^1$ and $Ar^2$ have the abovementioned meaning,
which are obtained with the aid of the processes (a), (b) (c) or (d), are reacted with halogenating or nitrating agents of the formula (VIII),
$R^1 - E^4$ (VIII)
in which
$R^1$ has the abovementioned meaning and
$E^4$ represents an electron-withdrawing leaving group, if appropriate in the presence of a diluent and if appropriate in the presence of a catalyst or reaction auxiliary.

4. Herbicidal agents, characterized in that they contain at least one 5-acylamido-1-aryl-pyrazole of the formula (I) according to claims 1 to 3.

5. Process for combating weeds, characterized in that 5-acylamido-1-aryl-pyrazoles of the formula (I), according to claims 1 to 3, are allowed to act on the weeds and/or on their habitat.

6. Use of 5-acylamido-1-aryl-pyrazoles of the formula (I), according to claims 1 to 3, for combating weeds.

7. Process for the preparation of herbicidal agents, characterized in that 5-acylamido-1-aryl-pyrazoles of the formula (I), according to claims 1 to 3, are mixed with extenders and/or surface-active substances.

**Revendications**

1. 5-acylamido-1-aryl-pyrazoles de formule générale (I)

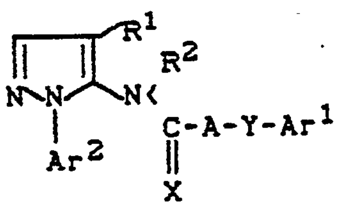

(I)

dans laquelle
$R^1$ est un groupe nitro, le fluor, le chlore, le brome ou l'iode,
$R^2$ représente l'hydrogène ou un groupe alkyle à chaîne droite ou ramifiée ayant 1 à 12 atomes de carbone,
X est l'oxygène ou le soufre,
Y est l'oxygène, le soufre, un groupe sulfinyle ou un groupe sulfonyle,
A désigne un pont alkylénique à chaîne droite ou ramifiée ayant 1 à 6 atomes de carbone, portant éventuellement un ou plusieurs substituants halogéno identiques ou différents,
$Ar^1$ désigne un groupe phényle, 2-pyridyle, 3-pyridyle, 4-pyridyle, 2-pyrimidyle, 4-pyrimidyle, 5-pyrimidyle, 3-pyridazinyle, 4-pyridazinyle, pyrazinyle ou s-triazinyle, chacun portant le cas échéant un ou plusieurs substituants identiques ou différents, et on considère alors dans chaque cas comme substituants: un halogène, un groupe cyano, nitro, un groupe alkyle, alkoxy ou alkoxycarbonyle chacun à chaîne droite ou ramifiée avec chacun 1 à 4 atomes de carbone dans les parties alkyle, un groupe halogénalkyle ou halogénalkoxy chacun à chaîne droite ou ramifiée avec 1 à 4 atomes de carbone et 1 à 9 atomes d'halogènes identiques ou différents, ou un reste $-S(O)_m-R^4$,où
$R^4$ désigne un groupe amino, un groupe alkyle, alkylamino, dialkylamino ou halogénalkyle, chacun à chaîne droite ou ramifiée avec 1 à 4 atomes de carbone dans les parties alkyle individuelles et, dans le cas du groupe halogénalkyle, avec 1 à 9 atomes d'halogènes identiques ou différents et
m est un nombre égal à 0, 1 ou 2, de même que
$Ar^2$ désigne un groupe phényle, 2-pyridyle, 3-pyridyle ou 4-pyridyle portant chacun le cas échéant un

ou plusieurs substituants identiques ou différents, et on considère alors comme substituants dans chaque cas ceux qui ont été mentionnés pour $Ar^1$.

2. 5-acylamido-1-aryl-pyrazoles de formule (I) suivant la revendication 1, dans lesquels

$R^1$ est un groupe nitro, le chlore ou le brome,

$R^2$ est l'hydrogène ou un groupe alkyle à chaîne droite ou à chaîne ramifiée ayant 1 à 8 atomes de carbone,

X est l'oxygène ou le soufre,

Y est l'oxygène, le soufre, un groupe sulfinyle ou un groupe sulfonyle,

A est un chaînon de pontage de formule: $-CH_2-$; $-CH_2-CH_2-$;

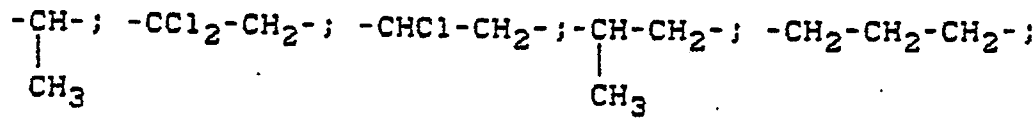

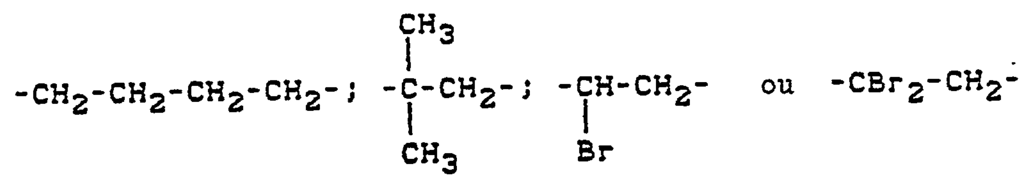

ou

$Ar^1$ désigne un groupe phényle, 2-pyridyle, 3-pyridyle, 4-pyridyle, 2-pyrimidyle, 4-pyrimidyle, 5-pyrimidyle, 3-pyridazinyle, 4-pyridazinyle, pyrazinyle ou s-triazinyle, chacun portant le cas échéant un à trois substituants identiques ou différents, et on considère alors comme substituants dans chaque cas: un groupe cyano, nitro, le fluor, le chlore, le brome, l'iode, un groupe méthyle, éthyle, n-propyle, isopropyle, n-butyle, isobutyle, sec.-butyle, tertio-butyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorométhyle, pentafluoréthyle, tétrafluoréthyle, trifluorochloréthyle, trifluoréthyle, difluorodichloréthyle, trifluorodichloréthyle, pentachloréthyle, trifluorométhoxy, trichlorométhoxy, dichlorofluorométhoxy, difluorochloromethoxy, chlorométhoxy, dichlorométhoxy, difluorométhoxy, pentafluoréthoxy, tétrafluoréthoxy, trifluorochloréthoxy, trifluoréthoxy, difluorodichloréthoxy, trifluorodichloréthoxy, pentachloréthoxy, ou un reste $-S(O)_m-R^4$, où

$R^4$ est un groupe amino, méthylamino, éthylamino, diméthylamino, diéthylamino, fluorodichlorométhyle, difluorométhyle, tétrafluoréthyle, trichloréthyle, trifluorométhyle, difluorochlorométhyle, méthyle ou éthyle et

m est le nombre 0, 1 ou 2, de même que

$Ar^2$ désigne un groupe phényle portant éventuellement 1 à 5 substituants identiques ou différents ou un groupe 2-pyridyle ou 4-pyridyle portant éventuellement chacun 1 à 4 substituants identiques ou différents, et on considère alors dans chaque cas comme substituants ceux qui sont mentionnés à propos de $Ar^1$.

3. Procédé de préparation de 5-acylamido-1arylpyrazoles de formule (I)

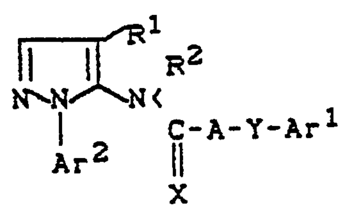

(I)

dans laquelle

$R^1$ est un groupe nitro, le fluor, le chlore, le brome ou l'iode,

$R^2$ représente l'hydrogène ou un groupe alkyle à chaîne droite ou ramifiée ayant 1 à 12 atomes de carbone,

X est l'oxygène ou le soufre,

Y est l'oxygène, le soufre, un groupe sulfinyle ou un groupe sulfonyle,

A désigne un pont alkylénique à chaîne droite ou ramifiée ayant 1 à 6 atomes de carbone, portant éventuellement un ou plusieurs substituants halogéno identiques ou différents,

$Ar^1$ désigne un groupe phényle, 2-pyridyle, 3-pyridyle, 4-pyridyle, 2-pyrimidyle, 4-pyrimidyle, 5-pyrimidyle, 3-pyridazinyle, 4-pyridazinyle, pyrazinyle ou s-triazinyle, chacun portant le cas échéant un ou plusieurs substituants identiques ou différents, et on considère alors dans chaque cas comme substituants: un halogène, un groupe cyano, nitro, un groupe alkyle, alkoxy ou alkoxycarbonyle chacun à chaîne droite ou ramifiée avec chacun 1 à 4 atomes de carbone dans les parties alkyle, un groupe halogénalkyle ou halogénalkoxy chacun à chaîne droite ou ramifié avec 1 à 4 atomes de carbone et 1 à 9 atomes d'halogènes identiques ou différents, ou un reste $-S(O)_m-R^4$, où

$R^4$ désigne un groupe amino, un groupe alkyle, alkylamino, dialkylamino ou halogénalkyle, chacun à chaîne droite ou ramifiée avec 1 à 4 atomes de carbone dans les parties alkyle individuelles et, dans le cas du groupe halogénalkyle, avec 1 à 9 atomes d'halogènes identiques ou différents et

m est un nombre égal à 0, 1 ou 2, de même que

$Ar^2$ désigne un groupe phényle, 2-pyridyle, 3-pyridyle ou 4-pyridyle portant chacun le cas échéant un ou plusieurs substituants identiques ou différents, et on considère alors comme substituants dans chaque cas ceux qui ont été mentionnés pour $Ar^1$,

caractérisé en ce que:

a) on obtient des 5-acylamido-1-aryl-pyrazoles de formule (Ia)

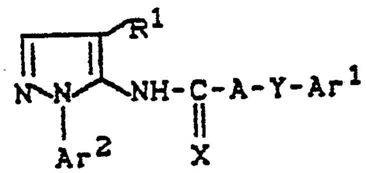

(Ia)

dans laquelle $R^1$, X, Y, A, $Ar^1$ et $Ar^2$ ont la définition indiquée ci-dessus, en faisant réagir des 5-amino-1-aryl-pyrazoles de formule (II)

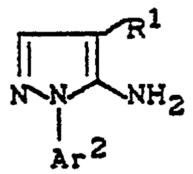

(II)

dans laquelle

$R^1$ et $Ar^2$ ont la définition indiquée ci-dessus, avec des agents d'acylation de formule (III)

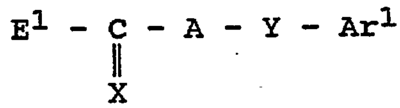

dans laquelle

X, Y, A et $Ar^1$ ont la définition indiquée ci-dessus et

$E^1$ est un groupe partant attirant les électrons, le cas échéant en présence d'un diluant, en la présence (éventuelle d'un accepteur d'acide ainsi que le cas échéant en présence d'un catalyseur, ou bien

b) on obtient des 5-acylamido-1-aryl-pyrazoles de formule (Ib)

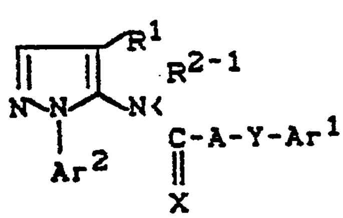

(Ib)

dans laquelle

1, X, Y, A, $Ar^1$ et $Ar^2$ ont la définition indiquée ci-dessus et

$R^{2-1}$ représente un groupe alkyle à chaîne droite ou ramifiée ayant 1 à 12 atomes de carbone, en faisant réagir les 5-acylamido-1-aryl-pyrazoles, obtenus conformément au procédé (a), de formule (Ia)

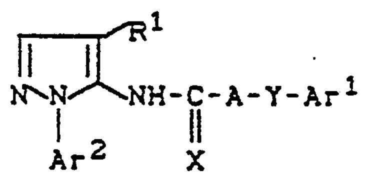

(Ia)

dans laquelle
$R^1$, X, Y, A, $Ar^1$ et $Ar^2$ ont la définition mentionnée cidessus, avec des agents d'alkylation de formule (IV)
$E^2 - R^{2-1}$(IV)
dans laquelle
$R^{2-1}$ a la définition indiquée ci-dessus et
$E^2$ est un halogène, un groupe alkoxysulfonyloxy éventuellement substitué ou un groupe arylsulfonyloxy éventuellement substitué, le cas échéant en présence d'un diluant et en la présence éventuelle d'un accepteur d'acide ainsi que, le cas échéant, en présence d'un catalyseur;
ou bien
c) on obtient des 5-acylamido-1-aryl-pyrazoles de formule (I) en faisant réagir des 5-acylamido-1-acylpyrazoles de formule (V)

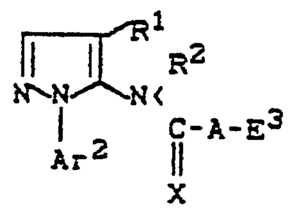

(V)

dans laquelle
$R^1$, $R^2$, X, A et $Ar^2$ ont la définition indiquée ci-dessus, et
$E^3$ représente un halogène, avec des dérivés phénoliques ou thiophénoliques de formule (VI)
$Ar^1 - Y - Z$ (VI)
dans laquelle
$Ar^1$ et Y ont la définition indiquée ci-dessus et
Z représente l'hydrogène ou un équivalent d'un cation de métal alcalino-terreux ou alcalin, éventuellement en présence d'un diluant et, le cas échéant, en présence d'un accepteur d'acide, et en la présence éventuelle d'un catalyseur approprié, ou bien
d) on obtient des 5-acylamido-1-aryl-pyrazoles de formule (Ic)

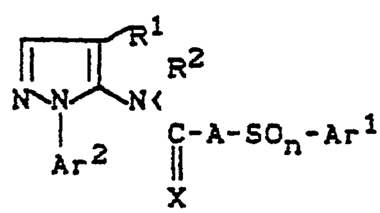

(Ic)

dans laquelle
$R^1$, $R^2$, X, A, $Ar^1$ et $Ar^2$ ont la définition indiquée ci-dessus et
n représente le nombre 1 ou 2, en faisant réagir les 5-acylamido-1-aryl-pyrazoles, obtenus à l'aide des procédés (a), (b) ou (c), de formule (Id)

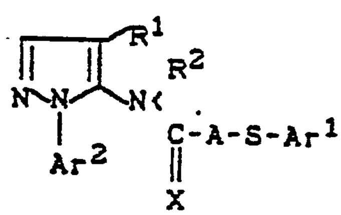

(Id)

dans laquelle.
$R^1$, $R^2$, X, A, $Ar^1$ et $Ar^2$ ont la définition indiquée ci-dessus,

avec des agents oxydants de formule (VII)

$R^3-O-O-H$ (VII)

dans laquelle

$R^3$ représente l'hydrogène ou un groupe alcanoyle ou aroyle chacun éventuellement substitué, le cas échéant en présence d'un diluant et en la présence éventuelle d'un accepteur d'acide, ainsi que, le cas échéant, en présence d'un catalyseur, ou bien

e) on obtient des 5-acylamido-1-aryl-pyrazoles de formule (Ie)

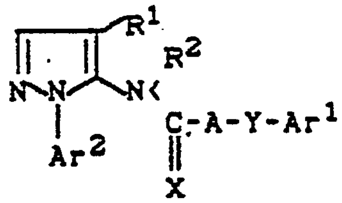

(Ie)

dans laquelle

$R^2$, X, A, Y, $Ar^1$ et $Ar^2$ ont la définition indiquée ci-dessus et

$R^1$ représente un groupe nitro, le fluor, le chlore, le brome ou l'iode, en faisant réagir les 5-acylamido-1-aryl-pyrazoles obtenus à l'aide des procédés (a), (b), (c) ou (d), de formule (If)

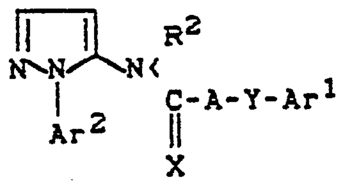

(If)

dans laquelle

$R^2$, X, Y, A, $Ar^1$ et $Ar^2$ ont la définition indiquée ci-dessus avec des agents d'halogénation ou de nitration de formule (VIII)

$R^1-E^4$ (VIII)

dans laquelle

$R^1$ a la définition indiquée ci-dessus et

$E^4$ est un groupe partant attirant les électrons, éventuellement en présence d'un diluant et le cas échéant en présence d'un catalyseur ou d'une substance auxiliaire de réaction.

4. Compositions herbicides, caractérisées par une teneur en au moins un 5-acylamido-1-aryl-pyrazole de formule (I) suivant les revendications 1 à 3.

5. Procédé pour combattre des mauvaises herbes, caractérisé en ce qu'on fait agir des 5-acylamido-1-aryl-pyrazoles de formule (I) suivant les revendications 1 à 3 sur les mauvaises herbes et/ou sur leur milieu.

6. Utilisation de 5-acylamido-1-aryl-pyrazoles de formule (I) suivant les revendications 1 à 3 pour combattre des mauvaises herbes.

7. Procédé de préparation de compositions herbicides, caractérisé en ce qu'on mélange des 5-acylamido-1-aryl-pyrazoles de formule (I) suivant les revendications 1 à 3 avec des diluants et/ou des substances tensioactives.